# EUROPEAN PATENT APPLICATION

(11) **EP 3 211 076 A1**
(43) Date of publication of application: **30.08.2017**
(21) Application number: 14904623.7
(22) Date of filing: 20.10.2014
(51) Int. Cl.: C12N 9/24, C12R 1/66, C12R 1/69

(54) **NOVEL ALPHA-GLUCOSIDASE**

(71) Applicant: Showa Sangyo Co., Ltd., Tokyo 101-8521 (JP)
(72) Inventor: KAWANO, Atsushi, Tokyo 101-8521 (JP); YASUTAKE, Nozomu, Tokyo 101-8521 (JP); TERADA, Atsushi, Tokyo 101-8521 (JP); MATSUMOTO, Yuji, Tokyo 101-8521 (JP); TOMINAGA, Akihiro, Tokyo 101-8521 (JP)
(74) Representative: De Clercq & Partners
(86) International application number: PCT/JP2014/077849
(87) International publication number: WO 2016/063331

(57) **Abstract**

The problem to be solved by the invention is to provide a novel α-glucosidase exhibiting catalyzing the formation of saccharides containing repeating α-1,6 linkages and that has a high thermal stability.

α-Glucosidase was discovered in one type of genus *Aspergillus*, specifically *Aspergillus sojae.* This α-glucosidase was found to act on various α-oligosaccharides and α-polysaccharides and to exhibit high activity of generating saccharide containing repeating α-1,6 linkages. Furthermore, this α-glucosidase was found to have a high thermal stability in which at least 90% of the activity remains after incubation of the enzyme at 50°C for 1 h.

SEQUENCE LISTING

<110>  Showa Sangyo Co., Ltd.

<120>  Novel alpha-glucosidase

<130>  FA1625-14145

<160>  10

<170>  PatentIn version 3.5

<210>  1
<211>  3041
<212>  DNA
<213>  Aspergillus sojae

<400>  1
atgtatctta agaagctgct cacttcggct gcagccctga gtggctccgt ctttgcccag      60

agcaaggatg gggtagagga tctcgatggc cccggcgatg acctatacgt caaggacctt      120

tctggatgcc caggatacaa ggccacaaaa cactggcaga ctcggtctgg cttttatgcg      180

gacctgactc tcgcaggccc ggcatgcaac gtcttcggca ctgatctccc cgacttgaag      240

ctggaagtcg agtatcagac cagcgatcga ctccacgtca agatcttgga caccaacaac      300

actgtctacc aggttccaga cagcgttttc ccgcgtcctg ggtttggcga atggtgttcc      360

ccgaaggaca gcaagctcaa gttcgacttc caagccgacc ccttctcgtt cacagtgtct      420

cgcactgata ccggcgaagt gctcttcgat accactggca acaagctggt tttcgagagt      480

caatatgtct acctgaagac acaccttccg cagaacccgc atctgtacgg tctgggcgag      540

cacagtgacg ccttcatgct caacacgacc aactacaccc ggaccatcta cacccgtgat      600

gcttacggaa cacctcaagg cgagaacttg tacggtgctc atccgatcta ctttgaccac      660

aggcagaccg ggactcacgg agtgttcctg ctcaactcca acggcatgga tatcttcatc      720

gacaacaacg ccacccagta ccttgagtac aacatcatcg gcggtgttct ggatttctac      780

ttcattgctg gaccctcccc acgtgacgtg gccattcagt atgctgagat cacccagacc      840

ccgttgatga ctccttattg gggacttggc taccaccaat gcaagtacgg ctaccaagac      900

gtctacgagg ttgctgctgt tgtggccaat tactcaacca acaacattcc cctcgagacg      960

atctggactg atattgacta catggaccgt cgtcgcatct tcaccattga cccagaacgg      1020

ttccctgcca acctgtacaa agaccttgtg gacactatcc atgcgaggga ccagcattac      1080

atcgtcatgg tcgatcccgc tgtgtattac aaggagtcta acccggcgct tgatgaaggt      1140

ctcaggtatg acatcttcat gaaggagaac aatggctctg aatatcaggg tgtcgtctgg      1200

gctggtccaa gtcacttccc ggattggttt caccctgact ctcagcagta ctggagtgag      1260

cagttcctgg cattcttcga cggaaccaac ggcccagaca tcgatgctct gtggatcgac      1320

atgaacgagc ccgcaaactt ctacaaccgc ccatacccag gcaacaacac cacccccgag      1380

aacttcgccg aagtggatgg tgaccctccc gcggccccag cagtcagaga tggcccggat 1440

gctcctatcc ctggcttccc ggccagcctg cagccaaact gggttcaggg caacgcgact 1500

gagaagcgct caactgctgc tgttgtcaaa cgtcaacgct cgcagtcccg ccgcaacctc 1560

ggcgctggtc actggaagtc ccctaagggc aaggtcgacc cacgtgctgg ctggcaaaac 1620

ggcaaacaga ccggttctgg ctgcggcccc aacgaatgca agggtctccc caaccgtcat 1680

ctcatcaggc cgccgtacat gatccagaac ggtgccggtc ccacgcttgc cgacagcact 1740

gccgacacgg atctcgtgca gagcggtgga tacgtccagt atgatactca caacctttac 1800

ggcgctatga tgtcctcgca ttcccacaat gctatgcgcg ctaggcgtcc ggatgaccgg 1860

gctttggtga ttaccaggag cacgtttgct ggttccggaa aggatgtgtc ccactggctt 1920

ggaggtaaga ccatcaaaat attgacagta gcaccaacaa cagcatgcta attattctgc 1980

agataatgta tccgggtggc tttggtacca gttatcgatc agccagatcc tgcagttcgc 2040

atccttgtac cagatccccg ttgtgggtcc tgacgtttgc ggcttcggtg gtaatgtcac 2100

ggagaccctc tgtgccaggt aaggactcaa atcacggttc cctctacaga acaacaacag 2160

tccaggctaa tacagtgcaa tagatgggca accctcggct ccttctacac attcttccgc 2220

aaccacgccg agatctacgc gaacccgcaa gaattctacc gctggcccac agtagctcaa 2280

gccgcccgca acggaatctc catcagatac cagctccgta agccactcac ccagtcttct 2340

cccaaacaac caagtcaaga atatactaac aatacccagt ggactacatc tacacagcca 2400

tctacaaaca gaaccaaacc ggtacacccg ccctaaaccc cctattcttc aactacccca 2460

acgaccccaa cacctacccc atcgacctcc aattcttcta cggagacggc atcctcgtct 2520

cccccgtcac agaggaaaac agcacaagcg tcaccttcta cctccccgac gacatcttct 2580

acgaatgggg caccggcaag cccgtccgcg gacagggcga atacgtctcc ctcgataaca 2640

tcgattacac tgacatcacg atccactata agggtggcat tgtctatccc cagcgtatcg 2700

agagcgcaaa caccactacc gctctccgcc agaagggctt caacattgtt gttgcgccgg 2760

gcttggatgg ccgtgctgag ggttcgcttt atcttgatga tggagtgtca gttgtgcagg 2820

atactgtgtc ggagattgat ttcgtttatg aaaatggcaa gcttaccatg acgggttcgt 2880

ttgagtatga agctggggtt ggtattgaga ccatcactgt tttgggtgtg gagagcaagc 2940

ccgagggtga tgaggatgtg gagtatgatg cggagaataa gaagctggtc aagcatgttg 3000

atgtgccttt gacgggcgag aatgagatta cgattctgtg a 3041

<210> 2
<211> 2856
<212> DNA
<213> Aspergillus sojae

<400>  2
atgtatctta agaagctgct cacttcggct gcagccctga gtggctccgt ctttgcccag      60

agcaaggatg gggtagagga tctcgatggc cccggcgatg acctatacgt caaggacctt      120

tctggatgcc caggatacaa ggccacaaaa cactggcaga ctcggtctgg cttttatgcg      180

gacctgactc tcgcaggccc ggcatgcaac gtcttcggca ctgatctccc cgacttgaag      240

ctggaagtcg agtatcagac cagcgatcga ctccacgtca agatcttgga caccaacaac      300

actgtctacc aggttccaga cagcgttttc ccgcgtcctg ggtttggcga atggtgttcc      360

ccgaaggaca gcaagctcaa gttcgacttc caagccgacc ccttctcgtt cacagtgtct      420

cgcactgata ccggcgaagt gctcttcgat accactggca acaagctggt tttcgagagt      480

caatatgtct acctgaagac acaccttccg cagaacccgc atctgtacgg tctgggcgag      540

cacagtgacg ccttcatgct caacacgacc aactacaccc ggaccatcta cacccgtgat      600

gcttacggaa cacctcaagg cgagaacttg tacggtgctc atccgatcta ctttgaccac      660

aggcagaccg ggactcacgg agtgttcctg ctcaactcca acggcatgga tatcttcatc      720

gacaacaacg ccacccagta ccttgagtac aacatcatcg gcggtgttct ggatttctac      780

ttcattgctg gaccctcccc acgtgacgtg gccattcagt atgctgagat cacccagacc      840

ccgttgatga ctccttattg gggacttggc taccaccaat gcaagtacgg ctaccaagac      900

gtctacgagg ttgctgctgt tgtggccaat tactcaacca acaacattcc cctcgagacg      960

atctggactg atattgacta catggaccgt cgtcgcatct tcaccattga cccagaacgg      1020

ttccctgcca acctgtacaa agaccttgtg gacactatcc atgcgaggga ccagcattac      1080

atcgtcatgg tcgatcccgc tgtgtattac aaggagtcta acccggcgct tgatgaaggt      1140

ctcaggtatg acatcttcat gaaggagaac aatggctctg aatatcaggg tgtcgtctgg      1200

gctggtccaa gtcacttccc ggattggttt caccctgact ctcagcagta ctggagtgag      1260

cagttcctgg cattcttcga cggaaccaac ggcccagaca tcgatgctct gtggatcgac      1320

atgaacgagc ccgcaaactt ctacaaccgc ccatacccag gcaacaacac cacccccgag      1380

aacttcgccg aagtggatgg tgaccctccc gcggccccag cagtcagaga tggcccggat      1440

gctcctatcc ctggcttccc ggccagcctg cagccaaact gggttcaggg caacgcgact      1500

gagaagcgct caactgctgc tgttgtcaaa cgtcaacgct cgcagtcccg ccgcaacctc      1560

ggcgctggtc actggaagtc ccctaagggc aaggtcgacc cacgtgctgg ctggcaaaac      1620

ggcaaacaga ccggttctgg ctgcggcccc aacgaatgca agggtctccc caaccgtcat      1680

ctcatcaggc cgccgtacat gatccagaac ggtgccggtc ccacgcttgc cgacagcact      1740

gccgacacgg atctcgtgca gagcggtgga tacgtccagt atgatactca caacctttac      1800

ggcgctatga tgtcctcgca ttcccacaat gctatgcgcg ctaggcgtcc ggatgaccgg      1860

gctttggtga ttaccaggag cacgtttgct ggttccggaa aggatgtgtc ccactggctt 1920

ggagataatg tatccgggtg gctttggtac cagttatcga tcagccagat cctgcagttc 1980

gcatccttgt accagatccc cgttgtgggt cctgacgttt gcggcttcgg tggtaatgtc 2040

acggagaccc tctgtgccag atgggcaacc ctcggctcct tctacacatt cttccgcaac 2100

cacgccgaga tctacgcgaa cccgcaagaa ttctaccgct ggcccacagt agctcaagcc 2160

gcccgcaacg gaatctccat cagataccag ctcctggact acatctacac agccatctac 2220

aaacagaacc aaaccggtac acccgcccta aaccccctat tcttcaacta ccccaacgac 2280

cccaacacct accccatcga cctccaattc ttctacggag acggcatcct cgtctccccc 2340

gtcacagagg aaaacagcac aagcgtcacc ttctacctcc ccgacgacat cttctacgaa 2400

tggggcaccg gcaagcccgt ccgcggacag ggcgaatacg tctccctcga taacatcgat 2460

tacactgaca tcacgatcca ctataagggt ggcattgtct atccccagcg tatcgagagc 2520

gcaaacacca ctaccgctct ccgccagaag ggcttcaaca ttgttgttgc gccgggcttg 2580

gatggccgtg ctgagggttc gctttatctt gatgatggag tgtcagttgt gcaggatact 2640

gtgtcggaga ttgatttcgt ttatgaaaat ggcaagctta ccatgacggg ttcgtttgag 2700

tatgaagctg gggttggtat tgagaccatc actgttttgg gtgtggagag caagcccgag 2760

ggtgatgagg atgtggagta tgatgcggag aataagaagc tggtcaagca tgttgatgtg 2820

cctttgacgg gcgagaatga gattacgatt ctgtga 2856

<210> 3
<211> 951
<212> PRT
<213> Aspergillus sojae

<400> 3

Met Tyr Leu Lys Lys Leu Leu Thr Ser Ala Ala Ala Leu Ser Gly Ser
1               5                   10                  15

Val Phe Ala Gln Ser Lys Asp Gly Val Glu Asp Leu Asp Gly Pro Gly
            20                  25                  30

Asp Asp Leu Tyr Val Lys Asp Leu Ser Gly Cys Pro Gly Tyr Lys Ala
        35                  40                  45

Thr Lys His Trp Gln Thr Arg Ser Gly Phe Tyr Ala Asp Leu Thr Leu
    50                  55                  60

Ala Gly Pro Ala Cys Asn Val Phe Gly Thr Asp Leu Pro Asp Leu Lys
65                  70                  75                  80

Leu Glu Val Glu Tyr Gln Thr Ser Asp Arg Leu His Val Lys Ile Leu

85                  90                  95

Asp Thr Asn Asn Thr Val Tyr Gln Val Pro Asp Ser Val Phe Pro Arg
            100                 105                 110

Pro Gly Phe Gly Glu Trp Cys Ser Pro Lys Asp Ser Lys Leu Lys Phe
        115                 120                 125

Asp Phe Gln Ala Asp Pro Phe Ser Phe Thr Val Ser Arg Thr Asp Thr
    130                 135                 140

Gly Glu Val Leu Phe Asp Thr Thr Gly Asn Lys Leu Val Phe Glu Ser
145                 150                 155                 160

Gln Tyr Val Tyr Leu Lys Thr His Leu Pro Gln Asn Pro His Leu Tyr
                165                 170                 175

Gly Leu Gly Glu His Ser Asp Ala Phe Met Leu Asn Thr Thr Asn Tyr
            180                 185                 190

Thr Arg Thr Ile Tyr Thr Arg Asp Ala Tyr Gly Thr Pro Gln Gly Glu
        195                 200                 205

Asn Leu Tyr Gly Ala His Pro Ile Tyr Phe Asp His Arg Gln Thr Gly
    210                 215                 220

Thr His Gly Val Phe Leu Leu Asn Ser Asn Gly Met Asp Ile Phe Ile
225                 230                 235                 240

Asp Asn Asn Ala Thr Gln Tyr Leu Glu Tyr Asn Ile Ile Gly Gly Val
                245                 250                 255

Leu Asp Phe Tyr Phe Ile Ala Gly Pro Ser Pro Arg Asp Val Ala Ile
            260                 265                 270

Gln Tyr Ala Glu Ile Thr Gln Thr Pro Leu Met Thr Pro Tyr Trp Gly
        275                 280                 285

Leu Gly Tyr His Gln Cys Lys Tyr Gly Tyr Gln Asp Val Tyr Glu Val
    290                 295                 300

Ala Ala Val Val Ala Asn Tyr Ser Thr Asn Asn Ile Pro Leu Glu Thr
305                 310                 315                 320

Ile Trp Thr Asp Ile Asp Tyr Met Asp Arg Arg Ile Phe Thr Ile
                325                 330                 335

Asp Pro Glu Arg Phe Pro Ala Asn Leu Tyr Lys Asp Leu Val Asp Thr
            340                 345                 350

Ile His Ala Arg Asp Gln His Tyr Ile Val Met Val Asp Pro Ala Val
        355                 360                 365

Tyr Tyr Lys Glu Ser Asn Pro Ala Leu Asp Glu Gly Leu Arg Tyr Asp
    370                 375                 380

Ile Phe Met Lys Glu Asn Asn Gly Ser Glu Tyr Gln Gly Val Val Trp
385                 390                 395                 400

Ala Gly Pro Ser His Phe Pro Asp Trp Phe His Pro Asp Ser Gln Gln
                405                 410                 415

Tyr Trp Ser Glu Gln Phe Leu Ala Phe Phe Asp Gly Thr Asn Gly Pro
            420                 425                 430

Asp Ile Asp Ala Leu Trp Ile Asp Met Asn Glu Pro Ala Asn Phe Tyr
        435                 440                 445

Asn Arg Pro Tyr Pro Gly Asn Asn Thr Thr Pro Glu Asn Phe Ala Glu
    450                 455                 460

Val Asp Gly Asp Pro Pro Ala Ala Pro Ala Val Arg Asp Gly Pro Asp
465                 470                 475                 480

Ala Pro Ile Pro Gly Phe Pro Ala Ser Leu Gln Pro Asn Trp Val Gln
                485                 490                 495

Gly Asn Ala Thr Glu Lys Arg Ser Thr Ala Ala Val Val Lys Arg Gln
            500                 505                 510

Arg Ser Gln Ser Arg Arg Asn Leu Gly Ala Gly His Trp Lys Ser Pro
        515                 520                 525

Lys Gly Lys Val Asp Pro Arg Ala Gly Trp Gln Asn Gly Lys Gln Thr
    530                 535                 540

Gly Ser Gly Cys Gly Pro Asn Glu Cys Lys Gly Leu Pro Asn Arg His
545                 550                 555                 560

Leu Ile Arg Pro Pro Tyr Met Ile Gln Asn Gly Ala Gly Pro Thr Leu
                565                 570                 575

Ala Asp Ser Thr Ala Asp Thr Asp Leu Val Gln Ser Gly Gly Tyr Val
            580                 585                 590

Gln Tyr Asp Thr His Asn Leu Tyr Gly Ala Met Met Ser Ser His Ser
            595                 600                 605

His Asn Ala Met Arg Ala Arg Arg Pro Asp Asp Arg Ala Leu Val Ile
        610                 615                 620

Thr Arg Ser Thr Phe Ala Gly Ser Gly Lys Asp Val Ser His Trp Leu
625 630 635 640

Gly Asp Asn Val Ser Gly Trp Leu Trp Tyr Gln Leu Ser Ile Ser Gln
645 650 655

Ile Leu Gln Phe Ala Ser Leu Tyr Gln Ile Pro Val Val Gly Pro Asp
                660                 665                 670

Val Cys Gly Phe Gly Gly Asn Val Thr Glu Thr Leu Cys Ala Arg Trp
            675                 680                 685

Ala Thr Leu Gly Ser Phe Tyr Thr Phe Phe Arg Asn His Ala Glu Ile
        690                 695                 700

Tyr Ala Asn Pro Gln Glu Phe Tyr Arg Trp Pro Thr Val Ala Gln Ala
705 710 715 720

Ala Arg Asn Gly Ile Ser Ile Arg Tyr Gln Leu Leu Asp Tyr Ile Tyr
725 730 735

Thr Ala Ile Tyr Lys Gln Asn Gln Thr Gly Thr Pro Ala Leu Asn Pro
                740                 745                 750

Leu Phe Phe Asn Tyr Pro Asn Asp Pro Asn Thr Tyr Pro Ile Asp Leu
            755                 760                 765

Gln Phe Phe Tyr Gly Asp Gly Ile Leu Val Ser Pro Val Thr Glu Glu
        770                 775                 780

Asn Ser Thr Ser Val Thr Phe Tyr Leu Pro Asp Asp Ile Phe Tyr Glu
785 790 795 800

Trp Gly Thr Gly Lys Pro Val Arg Gly Gln Gly Glu Tyr Val Ser Leu
805 810 815

Asp Asn Ile Asp Tyr Thr Asp Ile Thr Ile His Tyr Lys Gly Gly Ile
                820                 825                 830

Val Tyr Pro Gln Arg Ile Glu Ser Ala Asn Thr Thr Thr Ala Leu Arg
            835                 840                 845

Gln Lys Gly Phe Asn Ile Val Val Ala Pro Gly Leu Asp Gly Arg Ala
    850                 855                 860

Glu Gly Ser Leu Tyr Leu Asp Asp Gly Val Ser Val Val Gln Asp Thr
865                 870                 875                 880

Val Ser Glu Ile Asp Phe Val Tyr Glu Asn Gly Lys Leu Thr Met Thr
                885                 890                 895

Gly Ser Phe Glu Tyr Glu Ala Gly Val Gly Ile Glu Thr Ile Thr Val
            900                 905                 910

Leu Gly Val Glu Ser Lys Pro Glu Gly Asp Glu Asp Val Glu Tyr Asp
        915                 920                 925

Ala Glu Asn Lys Lys Leu Val Lys His Val Asp Val Pro Leu Thr Gly
    930                 935                 940

Glu Asn Glu Ile Thr Ile Leu
945                 950

<210>  4
<211>  950
<212>  PRT
<213>  Aspergillus oryzae

<400>  4

Met His Leu Lys Lys Leu Leu Thr Ser Ala Ala Ala Leu Ser Gly Ser
1               5                   10                  15

Val Phe Ala Gln Ser Lys Asp Gly Val Glu Asp Leu Asp Gly Pro Gly
            20                  25                  30

Asp Asp Leu Tyr Val Lys Asp Leu Ser Gly Cys Pro Gly Tyr Lys Ala
        35                  40                  45

Thr Lys His Trp Gln Thr Arg Ser Gly Phe Tyr Ala Asp Leu Thr Leu
    50                  55                  60

Ala Gly Pro Ala Cys Asn Val Phe Gly Thr Asp Leu Pro Asp Leu Lys
65                  70                  75                  80

Leu Glu Val Glu Tyr Gln Thr Ser Asp Arg Leu His Val Lys Ile Leu
                85                  90                  95

Asp Thr Asn Asn Thr Val Tyr Gln Val Pro Asp Ser Val Phe Pro Arg
            100                 105                 110

Pro Gly Phe Gly Glu Trp Cys Ser Pro Lys Asp Ser Lys Leu Lys Phe
        115                 120                 125

Asp Phe Gln Ala Asp Pro Phe Ser Phe Thr Val Ser Arg Thr Asp Thr
    130                 135                 140

Gly Glu Val Leu Phe Asp Thr Thr Gly Asn Lys Leu Val Phe Glu Ser
145                 150                 155                 160

Gln Tyr Val Tyr Leu Lys Thr His Leu Pro Gln Asn Pro His Leu Tyr
                165                 170                 175

Gly Leu Gly Glu His Ser Asp Ala Phe Met Leu Asn Thr Thr Asn Tyr
            180                 185                 190

Thr Arg Thr Ile Tyr Thr Arg Asp Ala Tyr Gly Thr Pro Gln Gly Glu
        195                 200                 205

Asn Leu Tyr Gly Ala His Pro Ile Tyr Phe Asp His Arg Gln Thr Gly
    210                 215                 220

Thr His Gly Val Phe Leu Leu Asn Ser Asn Gly Met Asp Ile Phe Ile
225                 230                 235                 240

Asp Asn Asn Ser Thr Gln Phe Leu Glu Tyr Asn Ile Ile Gly Gly Val
                245                 250                 255

Leu Asp Phe Tyr Phe Ile Ala Gly Pro Thr Pro Arg Asp Val Ala Ile
            260                 265                 270

Gln Tyr Ala Glu Ile Thr Gln Thr Pro Leu Met Thr Pro Tyr Trp Gly
        275                 280                 285

Leu Gly Tyr His Gln Cys Lys Tyr Gly Tyr Gln Asp Val Tyr Glu Val
    290                 295                 300

Ala Ala Val Val Ala Asn Tyr Ser Thr Asn Asn Ile Pro Leu Glu Thr
305                 310                 315                 320

Ile Trp Thr Asp Ile Asp Tyr Met Asp Arg Arg Arg Ile Phe Thr Ile
                325                 330                 335

Asp Pro Glu Arg Phe Pro Ala Asp Leu Tyr Lys Asp Leu Val Asp Thr
            340                 345                 350

Ile His Ala Arg Asp Gln His Tyr Ile Val Met Val Asp Pro Ala Val

355                 360                 365

Tyr Tyr Lys Glu Ser Asn Pro Ala Leu Asp Glu Gly Leu Lys Tyr Asp
    370                 375                 380

Ile Phe Met Lys Glu Asn Asn Gly Ser Glu Tyr Gln Gly Val Val Trp
385                 390                 395                 400

Ala Gly Pro Ser His Phe Pro Asp Trp Phe His Pro Asp Ser Gln Gln
                405                 410                 415

Tyr Trp Ser Glu Gln Phe Leu Ala Phe Phe Asp Gly Thr Asn Gly Pro
            420                 425                 430

Asp Ile Asp Ala Leu Trp Ile Asp Met Asn Glu Pro Ala Asn Phe Tyr
        435                 440                 445

Asn His Pro Tyr Pro Gly Asn Asn Thr Thr Pro Glu Asn Phe Ala Glu
    450                 455                 460

Val Asp Gly Asp Pro Pro Ala Ala Pro Ala Val Arg Asp Gly Pro Asp
465                 470                 475                 480

Ala Pro Ile Pro Gly Phe Pro Ala Ser Leu Gln Pro Asn Trp Val Gln
                485                 490                 495

Gly Asn Ala Thr Glu Lys Arg Ser Thr Ala Ala Val Val Lys Arg Gln
            500                 505                 510

Arg Ser Gln Ser Arg Arg Asn Leu Gly Ala Gly His Trp Lys Ser Pro
        515                 520                 525

Lys Gly Lys Phe Asp Ala Arg Ala Gly Trp Gln His Gly Lys Gln Thr
    530                 535                 540

Gly Ser Gly Cys Gly Pro Asn Glu Cys Lys Gly Leu Pro Asn Arg His
545                 550                 555                 560

Leu Ile Arg Pro Pro Tyr Met Ile Gln Asn Gly Ala Gly Pro Thr Leu
                565                 570                 575

Ala Asp Ser Thr Ala Asp Thr Asp Leu Val Gln Ser Gly Gly Tyr Val
            580                 585                 590

Gln Tyr Asp Thr His Asn Leu Tyr Gly Ala Met Met Ser Ser His Ser
        595                 600                 605

His Asn Ala Met Arg Ala Arg Arg Pro Asp Asp Arg Ala Leu Val Ile

610                 615                 620

Thr Arg Ser Thr Phe Ala Gly Ser Gly Lys Asp Val Ser His Trp Leu
625                 630                 635                 640

Gly Asp Asn Val Ser Gly Trp Leu Trp Tyr Gln Leu Ser Ile Ser Gln
                645                 650                 655

Ile Leu Gln Phe Ala Ser Leu Tyr Gln Ile Pro Val Val Gly Pro Asp
            660                 665                 670

Val Cys Gly Phe Gly Gly Asn Val Thr Glu Thr Leu Cys Ala Arg Trp
        675                 680                 685

Ala Thr Leu Gly Ser Phe Tyr Thr Phe Phe Arg Asn His Ala Glu Ile
    690                 695                 700

Tyr Ala Asn Ser Gln Glu Phe Tyr Arg Trp Pro Thr Val Ala Gln Ala
705                 710                 715                 720

Ala Arg Asn Gly Ile Ser Ile Arg Tyr Gln Leu Leu Asp Tyr Ile Tyr
                725                 730                 735

Thr Ala Ile Tyr Lys Gln Asn Gln Thr Gly Thr Pro Ala Leu Asn Pro
            740                 745                 750

Leu Phe Phe Asn Tyr Pro Asn Asp Pro Asn Thr Tyr Pro Ile Asp Leu
        755                 760                 765

Gln Phe Phe Tyr Gly Asp Gly Ile Leu Val Ser Pro Val Thr Glu Glu
    770                 775                 780

Asn Ser Thr Ser Val Thr Phe Tyr Leu Pro Asp Asp Ile Phe Tyr Glu
785                 790                 795                 800

Trp Gly Thr Gly Lys Pro Val Arg Gly Gln Gly Glu Tyr Val Ser Leu
                805                 810                 815

Asp Asn Ile Asp Tyr Thr Asp Ile Thr Ile His Tyr Lys Gly Gly Ile
            820                 825                 830

Val Tyr Pro Gln Arg Ile Glu Ser Ala Asn Thr Thr Thr Ala Leu Arg
        835                 840                 845

Gln Lys Gly Phe Asn Ile Val Val Ala Pro Gly Leu Asp Gly Arg Ala
    850                 855                 860

Glu Gly Ser Leu Tyr Leu Asp Asp Gly Val Ser Val Val Gln Asp Thr
865                 870                 875                 880

Val Ser Glu Ile Asp Phe Val Tyr Glu Asn Gly Lys Leu Thr Met Thr
                885                 890                 895

Gly Ser Phe Glu Tyr Glu Ala Gly Val Gly Ile Glu Thr Ile Thr Val
            900                 905                 910

Leu Gly Val Glu Ser Lys Pro Glu Gly Asp Asp Val Glu Tyr Asp Ala
        915                 920                 925

Glu Asn Lys Lys Leu Val Lys His Val Asp Val Pro Leu Thr Gly Glu
    930                 935                 940

Asp Glu Ile Thr Ile Leu
945                 950

<210>  5
<211>  38
<212>  DNA
<213>  Artificial Sequence

<220>
<223>  primer

<400>  5
tgattacgcc aagcttgatt ttcactgtgg accagaca                              38

<210>  6
<211>  19
<212>  DNA
<213>  Artificial Sequence

<220>
<223>  primer

<400>  6
tttgaaggtg gtgcgaact                                                   19

<210>  7
<211>  41
<212>  DNA
<213>  Artificial Sequence

<220>
<223>  primer

<400>  7
cgcaccacct tcaaaatgta tcttaagaag ctgctcactt c                          41

<210>  8
<211>  23
<212>  DNA

<213>  Artificial Sequence

<220>
<223>  primer

<400>  8
cagaatcgta atctcattct cgc                                              23


<210>  9
<211>  69
<212>  DNA
<213>  Artificial Sequence

<220>
<223>  primer

<400>  9
gagattacga ttctgcacca ccaccaccac caccaccacc accactgaat gatttggttg    60

gtgagatag                                                              69


<210>  10
<211>  41
<212>  DNA
<213>  Artificial Sequence

<220>
<223>  primer

<400>  10
gtgaattcga gctcggtacc aggtgatgaa cggagcttta a                          41

## Description

### TECHNICAL FIELD

The present invention relates to a novel α-glucosidase. More specifically, it relates to a novel α-glucosidase derived from a genus *Aspergillus.*

### BACKGROUND ART

α-Glucosidase is an enzyme that catalyzes hydrolysis reaction by acting on an α-1,4 bond in saccharide, and it is widely distributed in the natural environment from microorganisms such as bacteria or filamentous fungi, to animals and plants. α-Glucosidase is also capable of catalyzing transglycosylation reaction, resulting in the production of oligosaccharides and polysaccharides which contain glycosidic bonds.

The following enzymes are classified as a α-Glucosidase having transglycosylation activity. For example, the enzyme from *Aspergillus nidulans* catalyzes the formation of isomaltose and panose (Non-Patent Document 1). The enzyme from the genus *Acremonium* catalyzes the formation of saccharides containing α-1,3 linkage (Patent Document 1). The enzyme from the genus *Aspergillus* catalyzes the formation of saccharides containing α-1,2 and α-1,3 linkage (Patent Document 2). The enzyme from *Lactobacillus rhamnosus* catalyzes the formation of saccharides containing α-1,6 linkage, resulting in the production of isomaltose (Patent Document 3). The enzyme from *Aspergillus niger* catalyzes the formation of saccharides containing α-1,6 linkage, resulting in the production of saccharides having one unit higher degree of polymerization than the original substrates. (Non-Patent Document 2), etc.

The following enzymes mostly from bacteria are classified as a α-Glucosidase catalyzing the formation of saccharides containing repeating α-1,6 linkages. Dextrin dextranase (Patent Documents 4, 5, 6 and 7), α-glucanotransferase (Patent Document 8), dextran glucanase (Patent Document 9), etc. However, all of these enzymes have low thermal stability, and they were insufficient for the industrial production of saccharides.

### CITATION LIST

### PATENT DOCUMENTS

[Patent Document 1] JP Publication No. H07-59559
[Patent Document 2] JP Patent No. 4830031
[Patent Document 3] JP Publication No.2011-223910
[Patent Document 4] JP Publication No. H04-293493
[Patent Document 5] JP Publication No. H05-236982
[Patent Document 6] JP Publication No. 2001-258589
[Patent Document 7] WO 2006/054474
[Patent Document 8] JP Publication No.2012-525840
[Patent Document 9] JP Publication No.2012-95606

### NON-PATENT DOCUMENTS

[Non-Patent Document 1] Applied and Environmental Microbiology, March 2002, 68:1250-1256
[Non-Patent Document 2] Carbohydrate Research, 1989, 185:147-162

### SUMMARY OF INVENTION

### TECHNICAL PROBLEM

The problem to be solved by the present invention is to provide a novel α-glucosidase catalyzing the formation of saccharides containing repeating α-1,6 linkages and that has a high thermal stability.

### SOLUTION TO PROBLEM

The present inventors performed extensive studies to solve the above problem and discovered novel α-glucosidase from *Aspergillus sojae* which is classified as a genus *Aspergillus.* They also discovered that this α-glucosidase exhibits a high activity toward various α-oligosaccharides and α-polysaccharides, resulting in the production of saccharides containing repeating α-1,6 linkages. They further discovered that this α-glucosidase has a high thermal stability in which 90% or more of the activity remains after incubation of the enzyme at 50°C for 1 h., and the invention was completed.

In other words, the present invention relates to the following matters without being limited thereby.
[1] α-Glucosidase from a genus *Aspergillus,* and that comprises enzymologic features shown below:
   (1) Action: react with saccharide selected from a group consisting of α-oligosaccharides and α-polysaccharides to form saccharide containing repeating α-1,6 linkages;
   (2) Substrate specificity: acts on maltose, isomaltose, maltotriose, isomaltotriose, maltotetraose, isomaltotetraose, maltopentaose, maltohexaose, maltoheptaose, amylose, soluble starch, dextran.
[2] The α-glucosidase according to [1] that further comprises enzymologic features shown below:
   (3) Molecular amount: 65,000 dalton and 76,000 dalton according to SDS-polyacrylamide gel electrophoresis;
   (4) Isoelectric point: pI 4.9 to 5.5 (central value 5.2)
   (5) Optimum temperature: 55°C for a 30 min., reaction at pH 6.0;
   (6) Optimum pH: pH 5.5 for a 30 min., reaction at 40°C;
   (7) Thermal stability: 90% or more of initial activity remains after incubated in pH5.0 at 50°C for 1hr
   (8) pH stability: 90% or more of initial activity remains after incubated in pH6.0 to 10.0 at 4°C for 24hr
[3] The α-glucosidase according to either [1] or [2] that is either (a) or (b) below:
   (a) α-glucosidase that consists of an amino acid sequence having at least 80% sequence identity with an amino acid sequence shown by SEQ ID NO: 3;
   (b) α-glucosidase that consists of an amino acid sequence in which 1 to 190 amino acids are lost, substituted or added to an amino acid sequence shown by SEQ ID NO: 3.
[4] α-Glucosidase that is either (a) or (b) below:
   (a) α-glucosidase that consists of an amino acid sequence having at least 80% sequence identity with an amino acid sequence shown by SEQ ID NO: 3, and that react with saccharide selected from a group consisting of α-oligosaccharides and α-polysaccharides to form saccharide containing repeating α-1,6 linkages;
   (b) α-glucosidase that consists of an amino acid sequence in which 1 to 190 amino acids are lost, substituted or added to an amino acid sequence shown by SEQ ID NO: 3, and that react with saccharide selected from a group consisting of α-oligosaccharides and α-polysaccharides to form saccharide containing repeating α-1,6 linkages.
[5] α-Glucosidase that was collected from a culture obtained by cultivating genus Aspergillus having nucleic acids of one of (a) to (e) below:
   (a) a nucleic acid containing a base sequence encoding protein that consists of an amino acid sequence having at least 80% sequence identity with an amino acid sequence shown by SEQ ID NO: 3, and that exhibits an activity of react with saccharide selected from a group consisting of α-oligosaccharides and α-polysaccharides to form saccharide containing repeating α-1,6 linkages;
   (b) a nucleic acid containing a base sequence encoding protein that consists of an amino acid sequence in which 1 to 190 amino acids are lost, substituted or added to an amino acid sequence shown by SEQ ID NO: 3, and that exhibits an activity of react with saccharide selected from a group consisting of α-oligosaccharides and α-polysaccharides to form saccharide containing repeating α-1,6 linkages;
   (c) a nucleic acid that hybridizes with a nucleic acid having a base sequence that is complementary to a base sequence encoding protein that consists of an amino acid shown by SEQ ID NO: 3 under a highly stringent condition, and that contains a base sequence encoding protein that exhibits an activity of react with saccharide selected from a group consisting of α-oligosaccharides and α-polysaccharides to form saccharide containing repeating α-1,6 linkages;
   (d) a nucleic acid that hybridizes with a nucleic acid having a base sequence that is complementary to a base sequence that consists of SEQ ID NO: 2, and that contains a base sequence encoding protein that exhibits an activity of react with saccharide selected from a group consisting of α-oligosaccharides and α-polysaccharides to form saccharide containing repeating α-1,6 linkages; and
   (e) a nucleic acid that consists of a base sequence having at least 75% sequence identity with a base sequence consisting of SEQ ID NO: 2, and that contains a base sequence encoding protein that exhibits an activity of react with saccharide selected from a group consisting of α-oligosaccharides and α-polysaccharides to form saccharide containing repeating α-1,6 linkages.
[6] A recombinant vector containing a nucleic acid that is one of (a) to (e) below:
   (a) a nucleic acid containing a base sequence encoding protein that consists of an amino acid sequence having at least 80% sequence identity with an amino acid sequence shown by SEQ ID NO: 3, and that exhibits an activity of react with saccharide selected from a group consisting of α-oligosaccharides and α-polysaccharides to form saccharide containing repeating α-1,6 linkages.;
   (b) a nucleic acid containing a base sequence encoding protein that consists of an amino acid sequence in which 1 to 190 amino acids are lost, substituted or added to an amino acid sequence shown by SEQ ID NO: 3, and that exhibits an activity of react with saccharide selected from a group consisting of α-oligosaccharides and α-polysaccharides to form saccharide containing repeating α-1,6 linkages.;
   (c) a nucleic acid that hybridizes with a nucleic acid having a base sequence that is complementary to a base sequence encoding protein that consists of an amino acid shown by SEQ ID NO: 3 under a highly stringent condition, and that contains a base sequence encoding protein that exhibits an activity of react with saccharide selected from a group consisting of α-oligosaccharides and α-polysaccharides to form saccharide containing repeating α-1,6 linkages.;
   (d) a nucleic acid that hybridizes with a nucleic acid having of a base sequence that is complementary to a base sequence consisting of SEQ ID NO: 2 under a highly stringent condition, and that contains a base sequence encoding protein that exhibits an activity of react with saccharide selected from a group consisting of α-oligosaccharides and α-polysaccharides to form saccharide containing repeating α-1,6 linkages;
   (e) a nucleic acid that consists of a base sequence having at least 75% sequence identity with a base sequence consisting of SEQ ID NO: 2, and that contains a base sequence encoding protein that exhibits an activity of react with saccharide selected from a group consisting of α-oligosaccharides and α-polysaccharides to form saccharide containing repeating α-1,6 linkages.
[7] A transformant obtained by transferring the recombinant vector according to [6].
[8] α-Glucosidase that was collected from a culture obtained by cultivating the transformant according to [7].
[9] The α-glucosidase according to any one of [1] to [5] and [8] containing an amino acid sequence of DALWIDMNEPANFX₁X₂X₃, wherein X₁X₂ is any amino acid, and X₃ is an amino acid other than histidine.
[10] The α-glucosidase according to [9], wherein X₁ is tyrosine and X₂ is asparagine.
[11] The α-glucosidase according to any one of [1] to [5] and [8] to [10], wherein the saccharide has repeating α-1,6 linkages containing at least three repeating α-1,6 linkages.
[12] The α-glucosidase according to any one of [1] to [5] and [8] to [11], wherein α-oligosaccharides are selected from a group consisting of maltose, kojibiose, nigerose, isomaltose, maltotriose, panose, isomaltotriose, maltotetraose, isomaltotetraose, maltopentaose, maltohexaose, maltoheptaose, α-cyclodextrine, β-cyclodextrine, and γ-cyclodextrine, and α-polysaccharides are selected from a group consisting of amylose, soluble starch, glycogen, and dextran.
[13] The α-glucosidase according to any one of [1] to [5] and [8] to [12] produced from a genus *Aspergillus* that belongs to genus *Aspergillus* section *Flavi.*
[14] The α-glucosidase according to [13] produced from *Aspergillus sojae* or *Aspergillus oryzae.*
[15] A method for producing α-glucosidase that comprises a step of cultivating a genus *Aspergillus* that contains a nucleic acid of one of (a) to (e) below to obtain a culture, and a step of collecting α-glucosidase from the culture:
   (a) a nucleic acid containing a base sequence encoding protein that consists of an amino acid sequence having at least 80% sequence identity with an amino acid sequence shown by SEQ ID NO: 3, and that exhibits an activity of react with saccharide selected from a group consisting of α-oligosaccharides and α-polysaccharides to form saccharide containing repeating α-1,6 linkages;
   (b) a nucleic acid containing a base sequence encoding protein that consists of an amino acid sequence in which 1 to 190 amino acids are lost, substituted or added to an amino acid sequence shown by SEQ ID NO: 3, and that exhibits an activity of react with saccharide selected from a group consisting of α-oligosaccharides and α-polysaccharides to form saccharide containing repeating α-1,6 linkages;
   (c) a nucleic acid that hybridizes with a nucleic acid consisting of a base sequence that is complementary to a base sequence encoding protein that consists of an amino acid shown by SEQ ID NO: 3 under a highly stringent condition, and that contains a base sequence encoding protein that exhibits an activity of react with saccharide selected from a group consisting of α-oligosaccharides and α-polysaccharides to form saccharide containing repeating α-1,6 linkages;
   (d) a nucleic acid that hybridizes with a nucleic acid consisting of a base sequence that is complementary to a base sequence consisting of SEQ ID NO: 2 under a highly stringent condition, and that contains a base sequence encoding protein that exhibits an activity of react with saccharide selected from a group consisting of α-oligosaccharides and α-polysaccharides to form saccharide containing repeating α-1,6 linkages;
   (e) a nucleic acid that consists of a base sequence having at least 75% sequence identity with a base sequence consisting of SEQ ID NO: 2, and that contains a base sequence encoding protein that exhibits an activity of react with saccharide selected from a group consisting of α-oligosaccharides and α-polysaccharides to form saccharide containing repeating α-1,6 linkages.
[16] A method for producing α-glucosidase that comprises a step of cultivating a transformant that has been transformed by a recombinant vector containing a nucleic acid of one of (a) to (e) below to obtain a culture, and a step of collecting α-glucosidase from that culture:
   (a) a nucleic acid containing a base sequence encoding protein that consists of an amino acid sequence having at least 80% sequence identity with an amino acid sequence shown by SEQ ID NO: 3, and that exhibits an activity of react with saccharide selected from a group consisting of α-oligosaccharides and α-polysaccharides to form saccharide containing repeating α-1,6 linkages;
   (b) a nucleic acid containing a base sequence encoding protein that consists of an amino acid sequence in which 1 to 190 amino acids are lost, substituted or added to an amino acid sequence shown by SEQ ID NO: 3, and that exhibits an activity of react with saccharide selected from a group consisting of α-oligosaccharides and α-polysaccharides to form saccharide containing repeating α-1,6 linkages;
   (c) a nucleic acid that hybridizes with a nucleic acid consisting of a base sequence that is complementary to a base sequence encoding protein that consists of an amino acid shown by SEQ ID NO: 3 under a highly stringent condition, and that contains a base sequence encoding protein that exhibits an activity of react with saccharide selected from a group consisting of α-oligosaccharides and α-polysaccharides to form saccharide containing repeating α-1,6 linkages;
   (d) a nucleic acid that hybridizes with a nucleic acid consisting of a base sequence that is complementary to a base sequence consisting of SEQ ID NO: 2 under a highly stringent condition, and that contains a base sequence encoding protein that exhibits an activity of react with saccharide selected from a group consisting of α-oligosaccharides and α-polysaccharides to form saccharide containing repeating α-1,6 linkages;
   (e) a nucleic acid that consists of a base sequence having at least 75% sequence identity with a base sequence consisting of SEQ ID NO: 2, and that contains a base sequence encoding protein that exhibits an activity of react with saccharide selected from a group consisting of α-oligosaccharides and α-polysaccharides to form saccharide containing repeating α-1,6 linkages.
[17] The method according to either [15] or [16] that contains a sequence of DALWIDMNEPANFX₁X₂X₃, wherein X₁X₂ is any amino acid, and X₃ is an amino acid other than histidine, in an amino acid sequence encoding α-glucosidase.
[18] The method according to [17], wherein X₁ is tyrosine and X₂ is asparagine.
[19] The method according to any one of [15] to [18], wherein genus *Aspergillus* or the transformant is a genus *Aspergillus* that belongs to genus *Aspergillus* section *Flavi.*
[20] The method according to [19], wherein genus *Aspergillus* or the transformant is *Aspergillus sojae* or *Aspergillus oryzae.*
[21] The method of producing saccharide comprising reacting α-glucosidase according to any one of [1] to [5] and [8] to [14] to saccharide selected from a group consisting of an α-oligosaccharides and α-polysaccharides.

### ADVANTAGEOUS EFFECTS OF INVENTION

The α-glucosidase of the present invention exhibits a high activity of reacting with various saccharide to form saccharide containing repeating α-1,6 linkages. Digestion of saccharides that contain α-1,6 linkages is known to be slow, and digestion of saccharides with extended α-1,6 linkages is considered difficult (The American Journal of Clinical Nutrition, vol. 4, p. 279, 1956). Therefore, it is considered that the enzyme of the present invention will provide saccharide that takes time to be digested after it is taken in and that does not cause a quick increase in the blood sugar level. It is also known that some saccharide containing repeating α-1,6 linkages reaches the colon without being digested/absorbed at the small intestine and utilized by enteric bacterium such as bifidobacterium. Therefore, it is considered that the enzyme of the present invention may provide sugar that improves intestinal flora.

Further, an intake of α-glucosidase having α-1,6 transformation activity is reported to suppress sugar absorption, accelerate the growth of intestinal bifidobacterium, and increase the Bacteroides/Firmicutes ratio which results in alleviation of obesity (BMC Gastroenterology, vol. 13, p. 81, 2013). Hence, the α-glucosidase of the present invention may be directly taken as a supplement, etc.

In addition, the α-glucosidase of the present invention maintains a high residual activity even at a 50°C, which enables industrial production of saccharide at high temperatures.

### BRIEF DESCRIPTION OF DRAWINGS

Figure 1 shows a structure of a plasmid vector that incorporates putative α-glucosidase gene derived from *Aspergillus sojae* NBRC4239 strain.
Figure 2 shows native-polyacrylamide gel electrophoresis (left panel) and SDS-polyacrylamide gel electrophoresis (right panel) of the purified fraction of α -glucosidase derived from NBRC4239 strain.
Figure 3 shows effect of temperature on the enzyme activity of α-glucosidase derived from NBRC4239 strain.
Figure 4 shows effect of pH on the enzyme activity of α-glucosidase derived from NBRC4239 strain.
Figure 5 shows thermal stability of the enzyme activity of α-glucosidase derived from NBRC4239 strain.
Figure 6 shows pH stability of the enzyme activity of α-glucosidase derived from NBRC4239 strain.
Figure 7A shows time courses of the molecular weight distribution of the reaction product from maltose until 72 hours by transglucosidase L "Amano" .
Figure 7B shows time courses of the isomer compositions of di-, tri- and tetra-saccharides in the reaction product from maltose until 72 hours by transglucosidase L "Amano".
Figure 8A shows the time courses of the molecular weight distribution of the reaction product from maltose until 72 hours by the enzyme solution obtained from Aspergillus niger ATCC10254 strain which was prepared by a method of Japanese Patent No. 4830031.
Figure 8B shows time courses of the isomer compositions of di-, tri- and tetra-saccharides in the reaction product from maltose until 72 hours by the enzyme solution obtained from Aspergillus niger ATCC10254 strain which was prepared by a method of Japanese Patent No. 4830031.
Figure 9 shows chromatogram of the molecular weight distribution of the reaction product from maltose at 72 hours by α-glucosidase derived from NBRC4239 strain.
Figure 10 shows chromatogram of the isomer compositions of the reaction product from maltose at 72 hours by α-glucosidase derived from NBRC4239 strain.
Figure 11A shows the time courses of the molecular weight distribution of the reaction product from maltose until 72 hours by α-glucosidase from NBRC4239 strain.
Figure 11B shows time courses of the isomer compositions of di-, tri- and tetra-saccharides in the reaction product from maltose until 72 hours by α-glucosidase from NBRC4239 strain.
Figure 12 shows time courses of the compositions of tetra-saccharides and higher saccharides containing repeating unit with α-1,6 linkages from maltose until 72 hours by various enzymes.
Figure 13 shows H-NMR spectra of the fractionated pentasaccharides obtained from the reaction product at 72 hours by acting α-glucosidase derived from NBRC4239 strain with maltose.
Figure 14 shows chromatogram of the molecular weight distribution of the reaction product from maltopentaose at 72 hours by α-glucosidase derived from NBRC4239 strain.
Figure 15 shows time courses of the compositions of hexasaccharides and higher saccharides of the reaction product from maltopentaose until 72 hours by various enzymes.
Figure 16 shows time courses of the compositions of hexasaccharides and higher saccharides containing repeating α-1,6 linkages from maltose until 72 hours by various enzymes.
Figure 17 shows partial conserved sequence alignment of the various enzymes belonging to the GH31 family and that expresses a range of the 3 residues downstream point from near the active center.

### DESCRIPTION OF EMBODIMENTS

### <α-Glucosidase from genus Aspergillus in present invention>

The present invention relates to a thermal resistant α-glucosidase that reacting with saccharide selected from a group consisting of α-oligosaccharides and α-polysaccharides to form saccharide containing repeating α-1,6 linkages, and a method thereof.

α-Oligosaccharides in present invention are saccharides that are disaccharide to nonasaccharide and that contain α-glucosidic bond in the molecule. Examples of α-oligosaccharides include maltose, kojibiose, nigerose, isomaltose, trehalose, maltotriose, panose, isomaltotriose, maltotetraose, isomaltotetraose, maltopentaose, maltohexaose, maltoheptaose, α-cyclodextrine, β-cyclodextrine, γ-cyclodextrine, etc. without being limited thereby.

α-Polysaccharides in this invention are saccharides that are decasaccharide or higher and that contain α-glucosidic bond in the molecule. Examples of α-polysaccharides include glycogen, dextran, amylose, and soluble starch, etc. without being limited thereby.

The α-glucosidase of the present invention may form saccharides containing repeating α-1,6 linkages. Saccharide containing repeating α-1,6 linkages is saccharide containing two or more α-1,6 linkages in a sugar chain, and it includes saccharide consisting of α-1,6 linkages and other linkages as well as saccharide consisting only of α-1,6 linkages. The presence of repeating α-1,6 linkages in a saccharide may be determined by an evaluation approach using dextranase degradation, or an analysis by NMR or methylation (Journal of Biochemistry, vol. 55, column 205, 1964). Specifically, it is possible to determine whether the saccharide contains repeating α-1,6 linkage by the methods in the Examples. The α-glucosidase of the present invention may also form tetrasaccharide or a higher saccharide that has three or more repeating α-1,6 linkages. In addition, the α-glucosidase of the present invention may be applied to maltopentaose to form hexasaccharide and a higher saccharide that has a repeating α-1,6 linkage that content of 5% or higher, more preferably 8% or higher, and even more preferably 10% or higher.

The α-glucosidase of the present invention is a thermal resistant enzyme having a residual activity of 90% or higher even after it is incubated at 50°C for 1 h.

The α-glucosidase of the present invention may be obtained, for example, from a filamentous fungi belonging to the genus *Aspergillus* (referred to hereinafter as a genus *Aspergillus*) as an enzyme having enzymologic features of:
(1) Action: react with saccharide selected from a group consisting of α-oligosaccharides and α-polysaccharides to form saccharide having repeating α-1,6 linkage;
(2) Substrate specificity: acts on maltose, isomaltose, maltotriose, isomaltotriose, maltotetraose, isomaltotetraose, maltopentaose, maltohexaose, maltoheptaose, amylose, soluble starch, dextran.

The α-glucosidase of the present invention may be enzymes further comprising following enzymologic features:
(3) Molecular amount: 65,000 dalton and 76,000 dalton according to SDS-polyacrylamide gel electrophoresis;
(4) Isoelectric point: pI 4.9 to 5.5 (central value 5.2);
(5) Optimal temperature: 55°C for a 30 min., reaction at pH 6.0;
(6) Optimal pH: pH 5.5 for a 30 min., reaction at 40°C;
(7) Thermal stability: 90% or more of initial activity remains at 50°C for pH 6.0, maintained for 1 h.;
(8) pH stability: 90% or more of initial activity remains at pH 6.0 to 10.0 for 4°C, maintained for 24 h.

The origin of genus *Aspergillus* to obtain the α-glucosidase of the present invention may preferably be genus *Aspergillus* belonging to genus *Aspergillus* section *Flavi.* Genus *Aspergillus* belonging to genus *Aspergillus* section *Flavi* may preferably be *Aspergillus sojae, Aspergillus oryzae,* or *Aspergillus flavus.* In addition, genus *Aspergillus* to obtain the α-glucosidase of the present invention may also preferably be *Aspergillus nidulans.*

The α-glucosidase of the present invention may be obtained by cultivate known method such as liquid cultivation and solid cultivation, and collecting enzyme having α-glucosidase activity by a commonly known method.

The carbon source of the culture medium for cultivation of the genus *Aspergillus* includes glucose, fructose, sucrose, lactose, starch, glycerol, dextrin, lecithin, etc. and these carbon sources were used alone or combined together. The nitrogen source of the culture medium for the cultivation includes both organic and inorganic. The organic nitrogen source of the culture medium for the cultivation includes peptone, yeast extract, soy bean, soy bean flour, rice bran, corn steep liquor, meat extract, casein and amino acid for example. The inorganic nitrogen source of the culture medium for the cultivation includes ammonium sulfate, ammonium nitrate, ammonium phosphate, diammonium phosphate, ammonium chloride, etc. Inorganic salts and trace minerals addition to the above culture medium may include salts of sodium, magnesium, potassium, iron, zinc, calcium or manganese, as well as vitamins, etc. Natural ingredients like a wheat bran, etc. may also be used as a culture medium containing each component described above.

Genus *Aspergillus* is generally cultivated at a temperature of 10 to 40°C, and it is preferred to advantageously adopt a cultivation temperature of 25 to 30°C, and to set the pH of the culture medium to 2.5 to 8.0. Although the required cultivation period differs by the fungus body concentration, pH of the culture medium, the culture medium temperature, and the component of the culture medium, cultivation is normally continue d for 3 to 9 days and it is terminated when the target α-glucosidase has reached its maximum.

After the genus *Aspergillus* is cultivated, the α-glucosidase of the present invention is collected. α-glucosidase activity of the present invention is observed in both the fungus body of the culture and the culture medium, and it may be purified by a commonly known method. In an exemplary case, the enzyme solution concentrated from a processed product of a culture is subjected to column chromatography, etc. and a fraction with high glucosidase activity is collected, then, a single purified α-glucosidase is obtained by collecting a single band from a native-polyacrylamide gel electrophoresis.

### <Amino Acid Sequence of α-Glucosidase of Present Invention>

The present invention provides α-glucosidase that consists of an amino acid sequence that has at least 80% sequence identity with an amino acid sequence shown by SEQ ID NO: 3, and having a enzymologic features described above. In addition, the present invention provides α-glucosidase that consists of an amino acid sequence having at least 80% sequence identity with an amino acid sequence shown by SEQ ID NO: 3, and reacting with saccharide selected from a group consisting of α-oligosaccharides and α-polysaccharides to form saccharide containing repeating α-1,6 linkages.

Specific examples include amino acid sequences having at least 80%, at least 90%, more preferably at least 95%, even more preferably at least 97% (e.g. 98%, further, 99%) sequence identity with an amino acid sequence shown by SEQ ID NO: 3.

The identity percentage of two amino acid sequences may be determined by visual tests and mathematical calculations. The identity percentage may also be determined using a computer program. Such computer programs include BLAST, FASTA (Altschul et al., J. Mol. Biol., 215:403-410 (1990)) and ClustalW (http://www.genome.jp/tools/clustalw/), etc. and default parameters may be applied. Furthermore, the various conditions (parameters) of identity search using the BLAST program are written in "Altschul et al. (Nucl. Acids. Res., 25, p.3389-3402, 1997)". It can be publicly obtained from the web sites of U.S. National Center of Biotechnology Information (NCB) or the DNA Data Bank of Japan (DDBJ) (Blast manual, Altschul et al. NCB/NLM/NIH Bethesda, MD 20894). It may also be determined using programs such as the genetic information processing software, GENETYX (produced by Genetyx), DNASIS Pro (produced by Hitachi Software), Vector NTI (produced by Infomax), etc.

The specific alignment scheme that arranges multiple amino acid sequences in parallel may match a specific short region, so it can detect a region having a significantly high sequence identity even if there is no significant relation between the full length sequences of the sequences that were used. Also, the BLAST algorism may use a BLOSUM 62 amino acid scoring matrix, in which the following parameters may be selected: (A) including a filter to mask a segment of query sequence having low composition complexity (determined by a SEG program by Wootton and Federhen (Computers and Chemistry, 1993); Wootton and Federhen, 1996 "Analysis of compositionally biased regions in sequence databases)" Methods Enzymol., 266: 544-71) or a segment consisting of a short cycled internal repeats (determined by a XNU program by Claverie and States (Computers and Chemistry, 1993)), and (B) expected probability of suitability found accidentally according to the threshold of statistical significance to report suitability against the database sequence or a statistical model of E-score (Karlin and Altschul, 1990); when the statistical significance from a specific suitability is larger than the E-score threshold, the suitability is not reported.

A protein consisting of an amino acid sequence having at least 80% sequence identity with the amino acid sequence shown by SEQ ID NO: 3 may be appropriately prepared by a commonly known method, including an exemplary method in which a single or multiple amino acids (e.g. 1 to 190, 1 to 90, 1 to 50, 1 to 30, 1 to 25, 1 to 20, 1 to 15, and more preferably 10, 9, 8, 7, 6, 5, 4, 3, 2, or 1) are lost, substituted or added to an amino acid sequence shown by SEQ ID NO: 3.

The Substitution described above is preferably conservative substitution. A conservative substitution is a substitution in which a specific amino acid residue is replaced with a residue having a similar physical/chemical feature, but it may be any kind of substitution as long as the feature relating to the structure of the original sequence is substantially intact, for example, if the substituted amino acid does not destroy the spiral existing in the original sequence, or if it does not destroy other types of secondary structure that characterize the original sequence.

The conservative substitution is generally achieved by introducing the residue by biological synthesis or chemical peptide synthesis, but chemical peptide synthesis is preferred. In this case, the substitute may include a non-natural amino acid residue, and includes reversed types of peptide mimics and amino acid sequences in which the non-substituted region is reversed, or inversed types in which the same region is inverted.

Shown below are amino acid residues categorized by substitutable residues, but substitutable amino acid residues are not limited to those shown below:
A Group: Leucine, isoleucine, norleucine, valine, norvaline, alanine, 2-amino butanoic acid, methionine, O-methylserine, t-butylglycine, t-butylalanine and cyclohexylalanine;
B Group: Aspartic acid, glutamic acid, isoaspartic acid, isoglutamic acid, 2-aminoadipic acid and 2-aminosuberic acid;
C Group: Asparagine and glutamine;
D Group: Lysine, arginine, ornithine, 2,4-diaminobutanoic acid and 2,3-diaminopropionic acid;
E Group: Proline, 3-hydroxyproline and 4-hydroxyproline;
F Group: Serine, threonine and homoserine;
G Group: Phenylalanine and tyrosine.

In a non-conservative substitution, one member of the above groups may be exchanged with a member of another group, but the hydropathy index of amino acid (hydropathy-amino acid index) should be considered to retain the biological function of the protein of the present invention (Kyte et al., J. Mol. Biol., 157:105-131(1982)).

Also, in a non-conservative substitution, the amino acid may be substituted based on hydrophilicity.

The names of bases and amino acids and abbreviations thereof in the present specification and drawings conform to the IUPAC-IUB Commission on Biochemical Nomenclature, or are based on conventional abbreviations in the field of art as described in Immunology--A Synthesis (ver. 2, E.S. Golub and D.R. Gren ed., Sinauer Associates, Sunderland MA (1991)). Also, if optical isomers exist for an amino acid, the L-isomer will be shown unless otherwise mentioned.

The stereoisomers of the above amino acid, such as the D-amino acid, non-natural amino acids such as α,α-disubstituted amino acid, N-alkyl amino acid, lactic acid and other non-conventional amino acids may also be elements that constitute the protein of the present invention.

A protein consisting of an amino acid sequence shown by SEQ ID NO: 3 as described above in which a single or multiple amino acids are lost, substituted or added to an amino acid sequence, may be prepared according to methods such as site-specific mutagenesis described in "Molecular Cloning, A Laboratory Manual 3rd ed." (Cold Spring Harbor Press (2001)), "Current Protocols in Molecular Biology" (John Wiley & Sons (1987-1997), "Kunkel (1985) Proc. Natl. Acad. Sci. USA 82: 488-92, Kunkel (1988) Method. Enzymol. 85: 2763-6." Preparation of mutants in which amino acids are lost, substituted or added to an amino acid sequence may be carried out by commonly known methods such as the Kunkel method or Gapped duplex method using mutation introducing kits using site-specific mutagenesis, such as QuikChangeTM Site-Directed Mutagenesis Kit (produced by Stratagene), GeneTailorTM Site-Directed Mutagenesis System (produced by Invitrogen), TaKaRa Site-Directed Mutagenesis System (Mutan-K, Mutan-Super Express Km, etc.: produced by Takara-Bio).

Preparation of mutants with retaining its activity in which the amino acid sequence of protein loses, substitutes or adds a single or multiple amino acids may be also carried out in addition to the method of site-specific mutagenesis described above by treating the gene at the mutation source, or a method of selectively cut the gene and removing, substituting or adding the selected nucleotides before ligating the gene.

Notation of a protein used in the present specification is illustrated by the standard method and the method conventionally used in the field of art, so that the amino acid terminal direction is to the left, while the carboxyl terminal direction to the right.

A person skilled in the art can design and create an appropriate mutant of α-glucosidase described in the present specification by a technology commonly known in the field of art. For example, altering the structure without harming the biological activity of the protein of the present invention may be carried out by considering a region that is not crucial to the biological activity of α-glucosidase in the present invention. An appropriate region in the protein molecule may be identified. In addition, residues and regions preserved between similar proteins may be identified. Also, a conservative amino acid may be introduced to a region that is crucial to the biological activity or structure of the protein of the present invention without harming the biological activity and without negatively affecting the polypeptide structure of protein.

Further, a protein consisting of an amino acid sequence that has at least 80% sequence identity with an amino acid sequence shown by SEQ ID NO: 3 may be isolated from a microorganism such as genus *Aspergillus,* or a microorganism having an amino acid sequence that has at least 80% sequence identity with an amino acid sequence shown by SEQ ID NO: 3. Its amino acid sequence is obtained by searching in a public sequence data base (e.g. NCBI database http://www.ncbi.nlm.nih.gov/). The protein consisting of an amino acid sequence that has at least 80% sequence identity with an amino acid sequence shown by SEQ ID NO: 3 may also be obtained by the nucleic acid encoding α-glucosidase that is cloned from genus *Aspergillus,* as described hereinafter. Its nucleic acid sequence is obtained by searching in a public sequence database (e.g. NCBI database http://www.ncbi.nlm.nih.gov/).

The present inventors discovered that it is important for the amino acid sequence encoding α-glucosidase of the present invention to have a sequence represented by "DALWIDMNEPANFX₁X₂X₃", wherein X₁X₂ is a random amino acid, and X₃ is an amino acid other than histidine, preferably arginine, is important for the activity of forming saccharides containing repeating α-1,6 linkages. It is preferable for X₁ to be tyrosine and X₂ to be asparagine. Hence, the mutant of the present invention may be any type of mutant as long as the above sequence is conserved and the above activity of the present invention is not harmed.

A person skilled in the art can perform the so-called structure-function study by identifying a similar peptide residue that is crucial in the biological activity or structure of the protein of the present invention, and comparing the amino acid residues of the peptides, and predicting which of the residue in the protein, this protein is similar to the present invention, is the amino acid residue corresponding to the amino acid residue crucial to the biological activity or structure. Furthermore, it is possible to select a mutant conserving the biological activity of the present invention by selecting the amino acid substitution. This substitution was selected by chemically similar amino acid to the predicted residue described above. In addition, a person skilled in the art can analyze the three dimensional structure and the amino acid sequence of the mutant of this protein. From the analysis, it is also possible to predict the amino acid residue alignment relating to the three dimensional structure of protein. The amino acid residue predicted as being on the protein surface may be responsible for an important interaction with other molecules, and a person skilled in the art may create a mutant without altering the amino acid residue predicted as being on the protein surface. In addition, a person skilled in the art may create a mutant having only one amino acid residue is substituted in the various amino acid residues of the protein at present invention. These mutants may be screened by a commonly known assay method and analyze each characteristic. Based on these information, it is possible to evaluate the availability of the amino acid by comparing a case in which the biological activity of a mutant in which a specific amino acid is substituted is lower than the biological activity of the present invention, a case in which no biological activity is exhibited, or a case in which an unsuitable activity is exhibited that inhibits the biological activity of the present protein. A person skilled in the art may readily evaluate an amino acid substitution that is undesirable as a mutant of the protein of the present invention by considering alone or combined with other mutant that is analyzed as described above.

### <Nucleic acid consisting of a base sequence encoding an amino acid sequence of the α-glucosidase of the present invention>

The present invention also provides α-glucosidase that is collected from a culture obtained by cultivating genus *Aspergillus* containing a nucleic acid which is one of (a) to (e) below:
(a) a nucleic acid containing a base sequence encoding a protein that consists of an amino acid sequence having at least 80% sequence identity with an amino acid sequence shown by SEQ ID NO: 3, and that exhibits an activity of reacting with saccharide selected from a group consisting of α-oligosaccharides and α-polysaccharides to form saccharide containing repeating α-1,6 linkages;
(b) a nucleic acid containing a base sequence encoding protein that consists of an amino acid sequence in which 1 to 190 amino acids are lost, substituted or added to an amino acid sequence shown by SEQ ID NO: 3, and that exhibits an activity of reacting with saccharide selected from a group consisting of α-oligosaccharides and α-polysaccharides to form saccharide containing repeating α-1,6 linkages;
(c) a nucleic acid that hybridizes with a nucleic acid consisting of a base sequence that is complementary to a base sequence encoding protein that consists of an amino acid represented by SEQ ID NO: 3 under a highly stringent condition, and that contains a base sequence encoding protein that exhibits an activity of reacting with saccharide selected from a group consisting of α-oligosaccharides and α-polysaccharides to form saccharide containing repeating α-1,6 linkages;
(d) a nucleic acid that hybridizes with a nucleic acid consisting of a base sequence that is complementary to a base sequence consisting of SEQ ID NO: 2 under a highly stringent condition, and that contains a base sequence encoding protein that exhibits an activity of reacting with saccharide selected from a group consisting of α-oligosaccharides and α-polysaccharides to form saccharide containing repeating α-1,6 linkages;
(e) a nucleic acid that consists of a base sequence having at least 75% sequence identity with an amino acid sequence consisting of SEQ ID NO: 2, and that contains a base sequence encoding protein that exhibits an activity of reacting with saccharide selected from a group consisting of α-oligosaccharides and α-polysaccharides to form saccharide containing repeating α-1,6 linkages.

A more detailed description is provided below.
(a) A nucleic acid containing a base sequence encoding a protein that consists of an amino acid sequence having at least 80% sequence identity with an amino acid sequence shown by SEQ ID NO: 3, and that exhibits an activity of reacting with saccharide selected from a group consisting of α-oligosaccharides and α-polysaccharides to form saccharide containing repeating α-1,6 linkages;
   The amino acid sequence having at least 80% sequence identity with an amino acid sequence shown by SEQ ID NO: 3 is as shown above. A person skilled in the art may appropriately determine the base sequence encoding such amino acid sequence.
(b) A nucleic acid containing a base sequence encoding protein that consists of an amino acid sequence in which 1 to 190 amino acids are lost, substituted or added to an amino acid sequence shown by SEQ ID NO: 3, and that exhibits an activity of reacting with saccharide selected from a group consisting of α-oligosaccharides and α-polysaccharides to form saccharide containing repeating α-1,6 linkages;
   The amino acid sequence in which 1 to 190 amino acids are lost, substituted or added to an amino acid sequence shown by SEQ ID NO: 3 may be an amino acid sequence consisting of an amino acid sequence modified from an amino acid sequence shown by SEQ ID NO: 3 by removing, substituting or adding 1 to 190, 1 to 90, 1 to 50, 1 to 30, 1 to 25, 1 to 20, 1 to 15, and more preferably 10, 9, 8, 7, 6, 5, 4, 3, 2 or 1 amino acid. A person skilled in the art may appropriately determine the base sequences that encode such amino acid sequences.
(c) A nucleic acid that hybridizes with a nucleic acid consisting of a base sequence that is complementary to a base sequence encoding protein that consists of an amino acid represented by SEQ ID NO: 3 under a highly stringent condition, and that contains a base sequence encoding protein that exhibits an activity of reacting with saccharide selected from a group consisting of α-oligosaccharides and α-polysaccharides to form saccharide containing repeating α-1,6 linkages.
   The above base sequence may be obtained from a cDNA library and a genome library, etc. by creating a probe by a method commonly known to a person skilled in the art using a suitable fragment, then using that probe in a commonly known hybridization method such as colony hybridization, plaque hybridization, and the southern blot method.
   Refer to "Molecular Cloning, A Laboratory Manual 3rd ed." (Cold Spring Harbor Press (2001); particularly, Section 6-7), "Current Protocols in Molecular Biology" (John Wiley & Sons (1987-1997); particularly, Section 6.3-6.4), "DNA Cloning 1: Core Techniques, A Practical Approach 2nd ed." (Oxford University (1995); particularly, Section2.10 for hybridization conditions) for details on the procedure of hybridization methods.
   The intensity of the hybridization condition is determined mainly by the hybridization condition, and more preferably by the hybridization condition and the washing condition.
   A high stringent condition includes hybridization and/or washing at a higher temperature and/or lower salt concentration than the medium stringent condition. For example, hybridization conditions include a condition of 0.1×SSC to 2×SSC, 55°C to 65°C, more preferably, a condition of 0.1×SSC to 1×SSC, 60°C to 65°C, and most preferably, a condition of 0.2×SSC, 63°C. Washing conditions include a condition of 0.2×SSC to 2×SSC, 50°C to 68°C, more preferably a condition of 0.2×SSC, and 60°C to 65°C.
   It is also possible to use a commercially available hybridization kit that does not use a radioactive substance for the probe. Specific examples include hybridization using a DIG nucleic acid detection kit (produced by Roche Diagnostics K.K.), or an ECL direct labeling & detection system (produced by Amersham).
(d) A nucleic acid that hybridizes with a nucleic acid consisting of a base sequence that is complementary to a base sequence consisting of SEQ ID NO: 2 under a highly stringent condition, and that contains a base sequence encoding protein that exhibits an activity of reacting with saccharide selected from a group consisting of α-oligosaccharides and α-polysaccharides to form saccharide containing repeating α-1,6 linkages.
   The hybridization condition is as shown above.
(e) A nucleic acid that consists of a base sequence having at least 75% sequence identity with an amino acid sequence consisting of SEQ ID NO: 2, and that contains a base sequence encoding protein that exhibits an activity of reacting with saccharide selected from a group consisting of α-oligosaccharides and α-polysaccharides to form saccharide containing repeating α-1,6 linkages.

The above base sequence includes base sequences that have 75% (e.g. at least 80%, more preferably at least 90%, even more preferably, 95%, 97% 98% or 99%) sequence identity to a base sequence consisting of SEQ ID NO: 2, and such example includes a base sequence encoding protein that exhibits the above activity of the present invention.

The identity percentage of the two nucleic acid sequences may be determined by the visual inspection or mathematical calculation, in which it is preferable to use a computer program to compare the sequence information of two nucleic acids for the determination process. Computer programs for comparing sequence include the BLASTN program (Altschul et al. (1990) J. Mol. Biol. 215: 403-10): Ver. 2.2.7 or WU BLASST 2.0 algorism that can be used from the website of the U.S. National Library of Medicine (http://www.ncbi.nlm.nih.gov/blast/bl2seq/bls.html). The standard default parameter of WU-BLAST 2.0 may be set to the parameter in the following web site (http://blast.wustl.edu).

The nucleic acid of any of (a) to (e) above may be cloned by screening from a cDNA library using a suitable probe. It may also be cloned by amplification through a PCR reaction using a suitable primer and being connected to an appropriate vector. It may further be sub-cloned to a different vector.

Commercially available plasmid vectors, such as pBlue-Script^{™}SK(+) (produced by Stratagene), pGEM-T (produced by Promega KK), p-Direct (produced by Clontech Laboratories, Inc [NOTE: currently Takara Bio USA, Inc.]), pCR2.1-TOPO (produced by Invitrogen Corporation), may be used. Also, with regards to the primer in an amplification by PCR reaction, the primer may be any section of the base sequence shown by the above SEQ ID NO: 2. The above primer and a heat resistant DNA polymerase, etc. are applied to cDNA prepared from a body of the genus *Aspergillus* to perform a PCR reaction. The above method conforms to "Molecular Cloning, A Laboratory Manual 3rd ed." (Cold Spring Harbor Press (2001)), and is readily performed by a person skilled in the art. The PCR reaction conditions of the present invention include the following conditions.

| | |
|---|---|
| Denaturation temperature: | 90 to 95°C |
| Annealing temperature: | 40 to 60°C |
| Elongation temperature: | 60 to 75°C |
| Cycle number: | 10 times or more |

The obtained PCR product may be purified by a commonly known method. Examples of such methods include methods using kits including GENECLEAN (Funakoshi Co., Ltd.), QIAquick PCR purification Kits (QIAGEN), ExoSAP-IT(GE Healthcare), a method using a DEAE-cellulose filter, and a method using dialysis tubes. When using an agarose gel, an agarose gel electrophoresis is performed and the base sequence fragment is cut out from the agarose gel to be purified by GENECLEAN (Funakoshi Co., Ltd.), QIAquick Gel extraction Kits (QIAGEN), or a freeze and squeeze method.

The base sequence of a cloned nucleic acid may be determined by a base sequencer.

### <Construction of Vectors for Expressing α-Glucosidase of the Present Invention and Preparation of a Transformant>

The present invention provides a recombinant vector containing a nucleic acid of any of (a) to (e). The present invention further provides a transformant that has been transformed by the above recombinant vector. The present invention further provides α-glucosidase collected from a culture obtained by cultivating the above transformant.

These recombinant vectors and transformants may be obtained by the following method. That is, a plasmid containing a nucleic acid that encodes α-glucosidase of the present invention is digested using a restricted enzyme. The restricted enzyme to be used includes EcoRI, KpnI, BamHI and SalI, without being limited thereby. Note that the terminal may be smoothed out by acting a T4 polymerase treatment. The digested base sequence fragment is purified by an agarose gel electrophoresis. The base sequence fragment may be incorporated into the expression vector by a commonly known method to obtain an α-glucosidase expression vector. The expression vector is introduced into the host to create a transformant, which is provided to induce the expression of the desired protein. A commercially available kit, such as the In Fusion HD Cloning Kit (produced by Clontech Laboratories, Inc [NOTE: currently Takara Bio USA, Inc.]) is used to build a recombinant vector. When doing above process, recombinant vector was constructed by introducing high expression promoter and terminator into pPTR II (Takara Bio, Inc.) digested by restricted enzymes KpnI, HindIII, combined with above α-glucosidase. The high expression promoter consists of promoter region of a translation elongation factor TEF1 derived from *Aspergillus oryzae* RIB40. The terminator consists of range from the terminator codon of the enzyme to 300 bp downstream of the terminator codon.

The expression vector and the host is not limited as long as they may express the desired protein, and possible hosts include eumycetes or bacteria, plants, animals and cells thereof. Possible eumycetes include filamentous fungi such as the genus *Aspergillus,* and yeast such as *Saccharomyces cerevisiae.* In addition, bacteria include *E. coli* and *Bacillus subtilis.* Plants include *Arabidopsis* and rice.

Methods for introducing recombinant vectors into filamentous fungi include the protoplast-PEG method (Agricultural and Biological Chemistry, vol. 51, p. 2549, 1987), the electroporation method, the spheroplast method, the particle delivery method, and a direct microinjection of DNA into the nucleus. A transformed strain may be obtained from an auxotrophic host strain by selecting a strain that grows in a selective culture that lacks that specific nutrient. A case using a drug resistant marker gene for transformation, a drug resistant cell colony may also be obtained by cultivating a strain in a selective culture.

Methods for introducing a recombinant vector into yeast include the lithium acetate method, the electroporation method, the spheroplast method, the dextran mediated transfection, precipitation of calcium phosphate, polybrene mediated transfection, the protoplast fusion, covering polynucleotide (single or multiple) in the liposome, and direct microinjection of DNA into the nucleus.

Electroporation and the calcium chloride method may be used as a method for introducing a recombinant vector into bacteria.

### <Method for α-Glucosidase production of Present Invention>

The present invention provides a method for producing α-glucosidase from a culture obtained by cultivating genus *Aspergillus* containing nucleic acid that includes a base sequence encoding α-glucosidase. In addition, the present invention provides a method for producing α-glucosidase from a culture obtained by cultivating a transformant that has been transformed by a recombinant vector containing nucleic acid that includes a base sequence encoding the above α-glucosidase.

Specifically, α-glucosidase may be produced by the following method. However, the production method is not limited to the given method, and other commonly known, general methods may also be used.

The culture medium for cultivating may be any culture solution (medium) as long as it has an appropriate pH, osmotic pressure, and the medium including required element for growing the hosts. Its element includes nutrient, minor components and biological materials such as serum or antibiotic. Examples of culture medium for cultivating genus *Aspergillus from* natural source, transformed genus *Aspergillus,* or yeast, include the YPD medium, YPD5 medium, SC-Trp medium, the Czapek-Dox agar or wheat bran, without being limited thereby. An example of a YPD medium is provided to show the composition of a specific medium: 10 g of yeast extract, 20 g of peptone, and 20 g of glucose per 1 liter. Microorganisms may be cultivated by being planted in a medium solution, or by being planted in a solid medium or semi-solid medium that incorporates agar, etc. as necessary.

The cultivation condition may be any condition as long as it is suitable for growing hosts and suitable for maintaining the produced enzyme at a stable state, but individual conditions such as the anaerobic property, the cultivating time, the temperature, the humidity, and the selection between a static culture and a shaking culture may be adjusted. The cultivation method may be includes under a single condition (1-step cultivation), 2-step or 3-step cultivation that have 2 or more different culture conditions.

The production method of α-glucosidase of the present invention is described using an example of *Aspergillus sojae.* A preculture solution of *Aspergillus sojae* is added to sterilized wheat bran and subjected to solid culture at 30°C for 4 days. Then, the wheat bran is washed with an MES buffer (pH 6.0), and filtered by Miracloth (produced by Merck Millipore Corporation), centrifuged at 3,500 rpm for 30 min., after which the supernatant is collected to obtain a crude enzyme solution. This crude enzyme solution may be used directly as the α-glucosidase of the present invention, or an appropriately purified enzyme may be used as the α-glucosidase of the present invention.

### <Production Method of Saccharide Using α-Glucosidase of the Present Invention>

The present invention relates to a production method of saccharide that includes acting the above α-glucosidase on saccharide selected from a group consisting of α-oligosaccharides and α-polysaccharides.

### EXAMPLES

The invention is described in detail based on the Examples. Note that the present invention is not limited to matters described in the following Examples.

### 1) Analysis Method of Enzyme Activity

### <Analysis Method of α-Glucosidase Activity>

The α-glucosidase activity of the enzyme was measured by a hydrolytic activity of the maltose as a substrate.

The hydrolytic activity using maltose as the substrate was measured as shown below. A 20 mM maltose (50µL) dissolved in the 20mM MES buffer (pH 6.0) and an enzyme solution diluted by the buffer are mixed and incubated at 40°C for 30 min., then, 1 µL of 10% oxalic acid solution was added and heated at 100°C for 10 min. to stop the reaction. The amount of liberated glucose in the reaction solution was measured by Glucose CII Test Wako (produced by Wako Pure Chemical Industries, Ltd.). One unit of α-glucosidase activity was defined as the amount of enzyme that catalyzes hydrolysis of 1 µmol of maltose per minute.

### 2) Analysis Method of Reaction Product from Applying Enzyme to Various Substrates

### <Analysis Method of Distribution of Degree of Polymerization of Reaction Products>

The degree of polymerization (DP) of the reaction product was analyzed by subjecting the reaction solution to the gel filtration column under the following conditions. HPLC Analysis Condition:
Column: MCI GEL CK04S (Mitsubishi Chemicals)
Column Temperature: 65°C
Mobile Phase Composition: Ultrapure Water
Detector: RI (differential refractometer)
Flow Rate: 0.35 mL/min.

### <Analysis Method of Isomer Composition of Reaction Product>

The composition of oligosaccharides containing α-1,2, α-1,3, α-1,4, α-1,6 linkages in the disaccharide, trisaccharide, and tetrasaccharide components of the reaction product was analyzed by subjecting the reaction solution to an amino column under the following conditions.

Each peak was identified by the comparison data with the elution time of the authentic saccharides that had been analyzed by the same condition, and the resulting amount was calculated from the peak area.
authentic saccharides: Commercially available reagents were used for maltose, kojibiose, nigerose, and isomaltose, which are disaccharides.

Maltotriose, isomaltotriose, and panose, which are trisaccharides, were identified using commercially available reagents, 3²-O-α-D-glucosyl-nigerose and 3²-O-α-D-glucosyl-maltose were identified by reference (Biochimica et Biophysica Acta, Vol 1700, p. 189, 2004)

With regards to tetrasaccharides, commercially available reagents were used for maltotetraose and isomaltotetraose, isomaltotriosyl glucose was identified by reference (The Japanese Society of Applied Glycoscience, vol. 53, p. 215, 2006), and 3²-O-α-nigerosyl-maltose, 3²-O-α-D-maltosyl-maltose, 4²-O-α-D-nigerosyl-maltose were identified by reference (Biochimica et Biophysica Acta, vol. 1700, p. 189, 2004).
HPLC Analysis Conditions:
Column: Unison UK-Amino 250×3 mm (Imtact Corp.)
Column Temperature: 50°C
Solvent Gradient:

**[Table 1]**

| Time(min) | acetonitrile | water |
|---|---|---|
| 0-30 | 88⇒81% | 12⇒19% |
| 30-65 | 81% | 19% |
| 65-75 | 60% | 40% |
| 75-88 | 88% | 12% |

Detector: NQAD Detector (Quant)
Flow Rate: 0.4 mL/min.

### <Analysis Method of Repeating α-1,6 Linkage Content in Reaction Product>

The repeating α-1,6 linkage content in the reaction product was measured by the simplified evaluation method using dextranase decomposition.

The Dextranase L "Amano" (Amano Enzyme Inc.) is known as an enzyme that decomposes dextran, which has repeating α-1,6 linkage. The dextranase derived from filamentous fungi, it is origin of Dextranase L "Amano", is known to decompose dextran to produce mainly isomaltose, isomaltotriose and a small amount of glucose (MICROBIOLOGY AND MOLECULAR BIOLOGY REVIEWS, Vol. 69, p. 306, 2005). The present inventors confirmed that Dextranase L "Amano" acts on the tetrasaccharide or higher saccharides having repeating α-1,6 linkage (isomaltotetraose, isomaltopentaose, isomaltohexaose, dextran) and produces components of trisaccharide or lower unit. Hence, the enzyme was applied to the reaction product obtained by applying α-glucosidase of the present invention to evaluate whether tetrasaccharide or higher saccharides containing repeating α-1,6 linkages was produced.

Specifically, 30 µL of the reaction solution (DS: solid concentration 30%), 30 µL of 1,000 fold dilution of Dextranase L "Amano," and 390 µL of a 20mM (pH 6.0) phosphate buffer were mixed and incubated at 50°C for 16 h., then the reaction was stopped by heating at 100°C for 10 min. Subsequently, the distribution of the degree of polymerization of the reaction solution not treated by dextranase and the reaction solution treated by dextranase were analyzed by <Analysis Method of Distribution of Degree of Polymerization of Reaction Products> described above. The decreased amount (Δ%) of the proportion of tetrasaccharide or higher saccharides in the reaction solution treated by dextranase against the proportion of tetrasaccharide or higher saccharides in the reaction solution not treated by dextranase was determined as the repeating α-1,6 linkage content of tetrasaccharide or higher saccharides.

### <Structural Analysis of Reaction Product>

The proportion of α-1,6 linkages included in the fraction of the reaction product was confirmed by the integral value of H-NMR.

The composition of the bond variations in the fraction of the reaction product was evaluated by methylation analysis (Journal of Biochemistry, Vol. 55, column 205, 1964).

### 3) Obtaining α-Glucosidase of the Present Invention

### <Definition of Gene Sequence of α-Glucosidase>

A sequence that has at least 90% sequence identity with the agdC that is putative α-glucosidase of the GH31 from *Aspergillus oryzae* was searched on the publicly disclosed genome sequences of the genus *Aspergillus,* and extracted from a genome sequence of the *Aspergillus sojae* NBRC4239 strain (Accession no. BACA00000000) (SEQ ID NO: 1).

### 4) Obtaining Gene Recombinant Strains of putative α-glucosidase from NBRC4239 Strain

### <Extraction of Chromosomal DNA>

*Aspergillus sojae* NBRC4239 strain was cultivated at 30°C for 24 h. using a 50ml content tube with 5 mL of sterilized YPD culture medium. Then, the culture fungus body was collected and extracted a chromosomal DNA with a DNeasy Plant Mini Kit (QIAGEN). The fungus body was destructed using a multi-beads shocker (Yasui Kikai Corporation) after an AP1 buffer, it is described above, was added.

### <Construction of Gene Recombinant Strain>

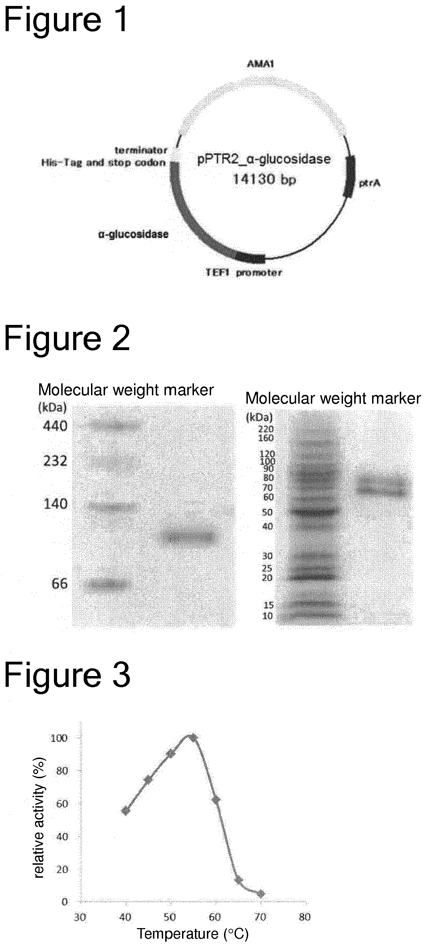

A primer set for obtaining a PCR product was designed in which a histidine tag of 10 residues was inserted in the front of the terminator codon of the defined gene sequence. Target gene was amplified by PCR with extracted chromosomal DNA as a template and the primer set described above. The PCR product, the promoter region of a translation elongation factor TEF1 derived from *Aspergillus oryzae* RIB40 which serves as a high expression promoter, and the terminator region with a range up to 300 bp downstream of the terminator codon of the enzyme, were inserted into KpnI and HindIII site of pPTR II (Takara Bio, Inc.) by using the In Fusion HD Cloning Kit (produced by Clontech Laboratories, Inc [NOTE: currently Takara Bio USA, Inc.]), The vector design is shown in Figure 1. Then, gene recombinant strain was constructed by protoplast-PEG method (Agricultural and Biological Chemistry, vol. 51, p. 2549, 1987) with *Aspergillus nidulans* ATCC38163 strain as a host.

The primers to amplify the promoter region of a translation elongation factor TEF1 derived from *Aspergillus oryzae* RIB40, the α-glucosidase gene, and the terminator region are shown as below.
Promoter F
   5'-TGATTACGCCAAGCTTGATTTTCACTGTGGACCAGACA-3'
Promoter R
   5'-TTTGAAGGTGGTGCGAACT-3'
α-Glucosidase F
   5'-CGCACCACCTTCAAAATGTATCTTAAGAAGCTGCTCACTTC-3'
α-Glucosidase R
   5 '-CAGAATCGTAATCTCATTCTCGC-3'
Terminator F (including Histidine tag) 
5‘-

GAGATTACGATTCTGCACCACCACCACCACCACCACCACCACCACTGAATGATTT

GGTTGGTGAGATAG-3’
Terminator R
   5 '-GTGAATTCGAGCTCGGTACCAGGTGATGAACGGAGCTTTAA-3'

### <Sequence Analysis of cDNA>

In order to confirm the cDNA sequence of the obtained enzyme, the fungus body was obtained by a similar procedure described at chromosome DNA extraction and RNA was extracted by RNeasy Plant Mini Kit (QIAGEN). The obtained RNA was subjected to reverse transcription to synthesize cDNA using Rever Tra Ace (Toyobo), and cDNA of the enzyme gene was obtained by PCR reaction. Sequence analysis was performed after the obtained cDNA was inserted into PCR2.1 TOPO by the TOPO TA Cloning Kit (both produced by Invitrogen), and the full length sequence of cDNA was confirmed (the section of SEQ ID NO: 2, histidine tag was excluded). The amino acid sequence of this enzyme was determined from the obtained cDNA (the section of SEQ ID NO: 3, histidine tag was excluded).

The amino acid sequence of the obtained enzyme had 99% sequence identity with the agdC that is putative α-glucosidase of the GH31 from *Aspergillus oryzae* (Accession No. XP_001825390). In the sequence of obtained enzyme, the two conserved regions, that is commonly shared by the GH31 family (PROSITE: PS00129, PS00707), were completely matched those of agdC. Therefore, the enzyme was considered belonging GH31 family.

### 5) Production/Purification of Transglycosidase derived from NBRC4239 Strain

### <Production of putative α-Glucosidase from NBRC4239 Strain>

The gene recombinant strain described above was cultivated at 37°C for 4 day with shaking, using a 2L Erlenmeyer flask with 1 L of Czapek-Dox broth and pyrithiamine. Then, the fungus was collected by Miracloth (produced by Merck Millipore Corporation), washed with distilled water, and squeezed well to remove water.

The mixture consist of 100 g of the obtained fungus body and 200 mL of 20 mM Tris buffer (pH 7.4) containing 0.5M NaCl and 20mM imidazole, were destructed at 20,000rpm for 30 sec. with Physcotron (Nichion Medical/Scientific Devices [NOTE: currently named Microtec Co., Ltd.]). The destructing process was repeated 5 times. Then, the mixture was centrifuged at 10,000 rpm for 20 min., and the supernatant was collected as crude enzyme solution. The activity of the crude enzyme solution was evaluated by the method described in the above <Analysis Method of α-Glucosidase Activity> and showed 97U for the activity. Thus, it was concluded that the enzyme has α-glucosidase activity.

### <Purification of α-Glucosidase derived from NBRC4239 Strain>

The obtained crude enzyme solution was subjected to a 2 step chromatographic separation.
(1) Ni-NTA Affinity Chromatography: A sample of the crude enzyme solution that was passed through a filter of 0.2 µm was passed through His-Trap HP (GE Health Care) and eluted with 20 mM Tris buffer (pH 7.4) containing 0.5 M NaCl and 500 mM imidazole. The histidine tagged protein was selectively collected by this process.
(2) Gel Filtration Chromatography: The eluate obtained in (1) was replaced with 20 mM MES buffer (pH 6.0) by ultrafiltration, and separated with Hiload 16/60 Superdex Prepgrade (GE Healthcare) to collect a fraction with a high α-glucosidase activity. The obtained fraction showed 17U for α-glucosidase activity.

The native-polyacrylamido gel electrophoresis (GE Healthcare, "PhastSystem") of the fraction obtained in (2) was shown in Figure 2 (left). It was a single band of 110,000 dalton. This result shows that obtained fraction was purified singularity.

### 6) Characterization of α-Glucosidase Derived from NBRC4239 Strain

Characterization of the enzyme was evaluated using the purified enzyme obtained in 5).

### <Molecular Weight of α-Glucosidase Derived from NBRC4239>

SDS-polyacrylamide gel electrophoresis (GE Healthcare, "PhastSystem") of the obtained purified enzyme was shown in Figure 2 (right). Two bands with molecular weight of 65,000 and 76,000 daltons were detected. By comparing this result with that of native-polyacrylamide electrophoresis, this enzyme was assumed to be a heterodimer structure.

Additionally, the isoelectric point of this enzyme was 4.9 to 5.5 (central value at 5.2) from the examination of isoelectric focusing electrophoresis (GE Healthcare, "PhastSystem").

<Optimum Temperature, Optimum pH>

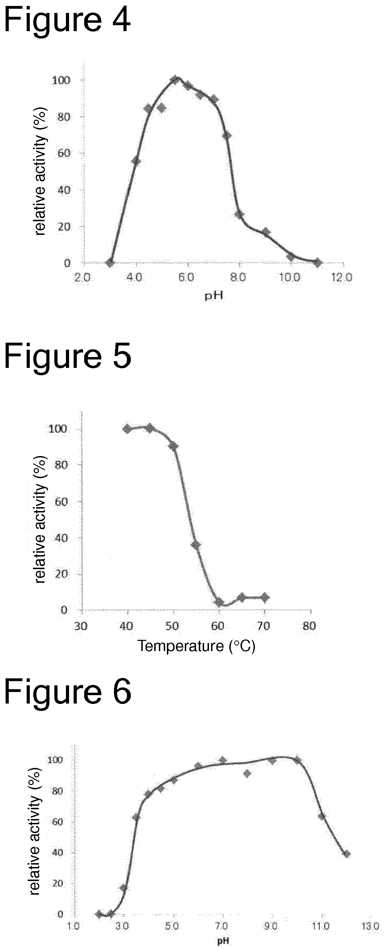

Effect of temperature and pH on the α-glucosidase activity of the enzyme was studied. The enzyme activity was analyzed according to <Analysis Method of α-Glucosidase Activity>. The results were shown in Figure 3 < Effect of Temperature> and Figure 4 <Effect of pH>. The optimum temperature of the present enzyme was 55°C on the reaction at pH 6.0 for 30 min, and the optimum pH was 5.5 on the reaction at 40°C for 30 min

Note that the effect of pH was evaluated using a phthalic acid buffer for a pH of 3.0 to 5.0, an MES buffer for a pH of 5.5 to 7.0 and MOPS buffer for a pH of 7.5 to 8.0, and a CAPS buffer for a pH of 9.0 to 11.0.

### <Thermal Stability, pH Stability>

Temperature and pH stability of the α-glucosidase activity of the present enzyme was studied. The enzyme activity was analyzed according to <Analysis Method of α-Glucosidase Activity>. The results were shown in Figure 5 < Thermal Stability> and Figure 6 <pH Stability>. The thermal stability of the present enzyme was 90% or more of the remaining activity after incubation of the enzyme at 50°C. The pH stability was 90% or more of the remaining activity in a range of pH 6.0 to 10.0.

To evaluate thermal stability, the enzyme solution (20 mM MES buffer, pH 6.0, containing CaCl₂) was incubated at each temperature for an hour and cooled by ice water, then the remaining enzyme activity was measured. To evaluate pH stability, the enzyme solution (40 mM buffer of each pH) was incubated at 4°C for 24 h. and the pH was adjusted to 6.0, then the remaining enzyme activity was measured.

Note that the pH Stability was evaluated using a phthalic acid buffer for a pH of 2.0 to 5.0, an MES buffer for a pH of 6.0 to 7.0 and MOPS buffer for a pH of 8.0, and a CAPS buffer for a pH of 9.0 to 12.0.

### <Substrate Specificity>

The substrate specificity of the present enzyme was evaluated at the conditions of 40°C and pH 6.0. The result was shown in Table 2 below.

**[Table 2]**

| Substrate Specificty | |
|---|---|
| Samples | Relative Activity* (%) |
| maltose (10mM) | 100 |
| kojibiose (10mM) | 91 |
| nigerose (10mM) | 153 |
| isomaltose (10mM) | 163 |
| trehalose (10mM) | 1 |
| maltotriose (10mM) | 45 |
| panose (10mM) | 153 |
| isomaltotriose (10mM) | 161 |
| maltotetraose (10mM) | 16 |
| isomaltotetraose (10mM) | 168 |
| maltopentaose (10mM) | 41 |
| maltohexaose (10mM) | 49 |
| maltoheptaose (10mM) | 57 |
| α-cyclodextrine (10mM) | 58 |
| β-cyclodextrine (10mM) | 48 |
| γ-cyclodextrine (10mM) | 14 |
| amylose (0.4%) | 55 |
| soluble starch (0.4%) | 135 |
| glycogen (0.4%) | 2 |
| dextran (0.4%) | 6 |

| | |
|---|---|
| * the decomposition rate of each substrate when the maltose decomposition rate for a substrate of 10 mM maltose is the substrate | |

The enzyme exhibited well hydrolytic activities and liberated glucose toward disaccharides such as maltose, kojibiose, nigerose and isomaltose, maltooligosaccharides having a degree of polymerization of 2 to 7 excluding maltotetraose, α-cyclodextrine, β-cyclodextrine, amylase and soluble starch.
In particular, the favorable substrate was isomaltooligosaccharide having a degree of polymerization of 2 to 4, which contains α-1,6 linkage.

Activities for maltotetraose and γ-cyclodextrine were a little low. Trehalose, glycogen and dextran were very slight.

### <Effect of Metal Ion>

The effect of various metal ions on the present enzyme was studied. The enzyme activity was analyzed according to <Analysis Method of α-Glucosidase Activity>. The result of an addition of ions to the reaction mixture was shown in Table 3 below.

The present enzyme increased its activity somewhat by calcium. Zinc ion, copper ion and ferric ion inhibited the activity. Sodium ion and EDTA also inhibited the activity somewhat.

**[Table 3]**

| Metal ion | Concentration (mM) | Specific Activity (%) |
|---|---|---|
| -- | | 100 |
| Ca²⁺ | 1 | 111 |
| Mg²⁺ | 1 | 96 |
| Na⁺ | 50 | 80 |
| Zn²⁺ | 1 | 8 |
| Cu²⁺ | 1 | 4 |
| Mn²⁺ | 1 | 105 |
| Fe²⁺ | 1 | 50 |
| Fe³⁺ | 1 | 15 |
| EDTA | 5 | 85 |

### 7) Comparison of α-Glucosidase Derived from NBRC4239 Strain and known Enzyme.

The activity of α-glucosidase derived from NBRC4239 strain and the enzyme mainly consist of known α-glucosidase, were compared using reaction products obtained by maltose (Degree of Polymerization, DP2) and maltopentaose (DP5) as a substrate.

### <Transglycosylation Product of Maltose (DP2) by Transglucosidase L "Amano">

A commercially available enzyme preparation Transglucosidase L "Amano" (Amano Enzyme) which has a main activity of α-glucosidase derived from the genus *Aspergillus* (*Aspergillus niger*), was added in an amount of 0.2 mL (1.8U/mL) to 0.4 mL of a 45% maltose solution (the final concentration 30%), and a reaction was continued at 40°C. After a predetermined time passed, the enzyme was inactivated by heating for 10 min using boiling water bath.

The distribution of the degree of polymerization of the obtained reaction product and the composition of isomers of the di-, tri- and tetra-saccharide components were analyzed according to the methods described in the above <Analysis Method of Distribution of Degree of Polymerization of Reaction Products> and <Analysis Method of Isomer Composition of Reaction Product>. Further, the content of repeating α-1,6 linkages in tetrasaccharide or a higher saccharide in the reaction product was analyzed according to the method described in the above <Analysis Method of Repeating α-1,6 Linkage Content in Reaction Product>.

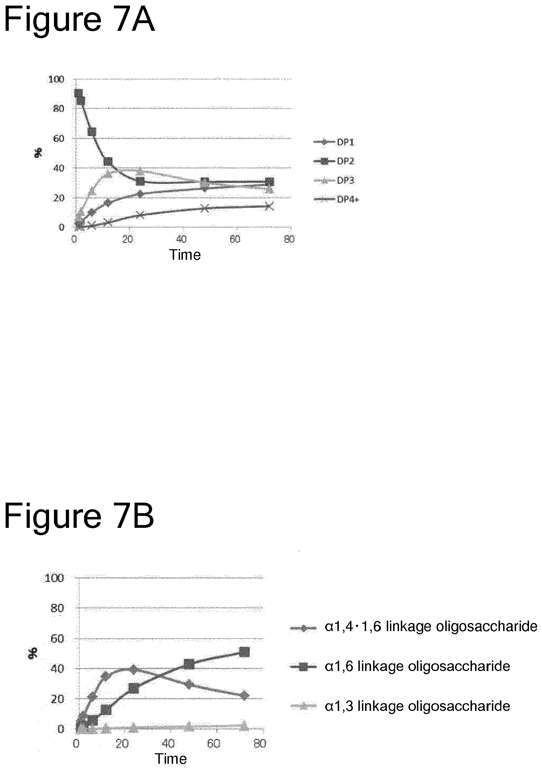

The time courses of the molecular weight distribution of the reaction products until 72 h. was shown in Figure 7A, and the time courses of the isomer compositions of di-, tri-and tetra-saccharides was shown in Figure 7B. In the distribution of the degree of polymerization, trisaccharide components were produced with the decrease of the substrate maltose (DP2), followed by an increase of components having even higher degree of polymerization. In the isomer composition of the di-, tri- and tetra-saccharide components, low molecular sugar containing both an α-1,4 bond and an α-1,6 linkage (panose, isomaltotriosyl glucose, hereinafter referred to as α-1,4•1,6 linkage oligosaccharides), subsequently, a low molecular sugar containing only α-1,6 linkage (isomaltose, isomaltotriose, isomaltotetraose, hereinafter referred to as α-1,6 linkage oligosaccharides) were increased.

The distribution of the molecular weight at 72 h. was monosaccharide 29.0%, disaccharide 30.9%, trisaccharide 25.9%, and tetrasaccharide or higher 14.2%. Furthermore, among the α-1,4•1,6 linkage oligosaccharides and the α-1,6 linkage oligosaccharides, a disaccharide isomaltose existed in an amount of 20.8%, panose and isomaltotriose, which are trisaccharides, respectively in amounts of 10.0% and 11.1%, and isomaltotriosyl glucose and isomaltotetraose, which are tetrasaccharides, respectively in amounts of 5.0% and 2.8%.

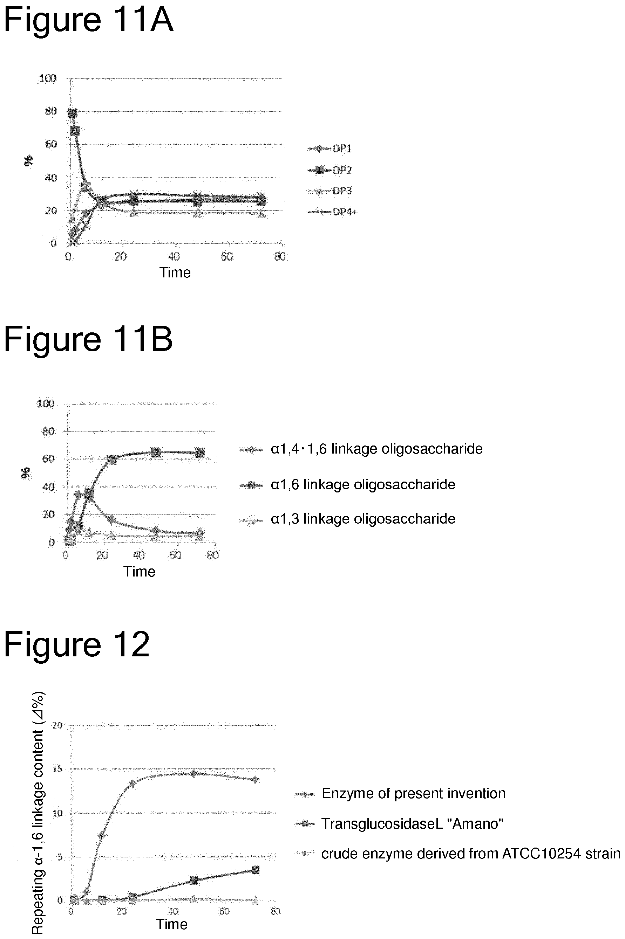

Time courses of the compositions of tetrasaccharides or higher saccharides containing repeating α-1,6 linkages until 72 h was shown in Figure 12. The content of repeating α-1,6 linkages in tetrasaccharide or a higher saccharide until 72 h was increased from 0% to 3%.

### <Transglycosylation Product of Maltose (DP2) by Crude Enzyme Solution Derived from Aspergillus niger ATCC10254 Strain>

A crude enzyme solution was obtained from an *Aspergillus niger* ATCC10254 strain, which is known to secrete α-glucosidase into the crude enzyme solution, by the method of Japanese Patent No. 4830031. 0.2 mL (1.8 U/mL) of the solution was added to 0.4 mL of a 45% maltose solution (final concentration 30%), and reaction was continued at 40°C. After a predetermined time passed, the solution was heated in the boiling bath for 10 min. to inactivate enzyme.

The distribution of the degree of polymerization of the obtained reaction product and the composition of isomers of the di-, tri- and tetra-saccharide components were analyzed according to the methods described in the above <Analysis Method of Distribution of Degree of Polymerization of Reaction Products> and <Analysis Method of Isomer Composition of Reaction Product>. Further, the content of repeating α-1,6 linkages in tetrasaccharide or a higher saccharide in the reaction product was analyzed according to the method described in the above <Analysis Method of Repeating α-1,6 Linkage Content in Reaction Product>.

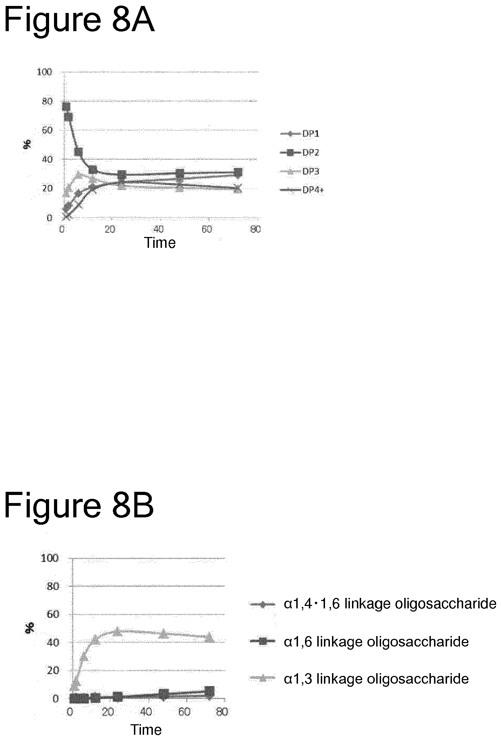

The time courses of the molecular weight distribution of the reaction products until 72 h. was shown in Figure 8A, and the time courses of the isomer compositions of di-, tri-and tetra-saccharides was shown in Figure 8B. In the distribution of degree of polymerization, trisaccharide components were generated with the decrease of the substrate maltose (DP2), then, the components having an even higher degree of polymerization increase. In the isomer composition of the di-, tri- and tetra-saccharide components, low molecular sugar containing an α-1,3 linkage (hereinafter referred to as α-1,3 linkage oligosaccharide) was increased at the initial stage of reaction, and reached equilibrium and maintained as it is.

The distribution of the molecular amount at 72 h. from the beginning of reaction was monosaccharide 29.2%, disaccharide 31.0%, trisaccharide 19.5%, and tetrasaccharide or higher 20.2%. Furthermore, among the α-1,4•1,6 linkage oligosaccharides and the α-1,6 linkage oligosaccharides, isomaltose which is a disaccharide existed in an amount of 3.2%, and panose which is a trisaccharide in an amount of 1.3%. Isomaltotriose, isomaltotriosyl glucose, and isomaltotetraose were not observed.

Time courses of the compositions of tetrasaccharides or higher saccharides containing repeating α-1,6 linkages until 72 h was shown in Figure 12. Tetrasaccharides or higher saccharides containing repeatingα-1,6 linkages were not observed until 72 h entirely.

### <Transglycosylation Product of Maltose (DP2) derived from NBRC4239 Strain>

A purified enzyme (0.2 mL (1.8 U/mL) obtained by the method shown in 5) was added to 0.4 mL of a 45% maltose solution (final concentration 30%) and incubated at 40°C. After a predetermined time passed, the solution was heated in the boiling bath for 10 min. to inactivate enzyme.

The distribution of the degree of polymerization of the obtained reaction product and the composition of isomers of the di-, tri- and tetra-saccharide components were analyzed according to the methods described in the above <Analysis Method of Distribution of Degree of Polymerization of Reaction Products> and <Analysis Method of Isomer Composition of Reaction Product>. Further, the content of repeating α-1,6 linkages in tetrasaccharide or a higher saccharide in the reaction product was analyzed according to the method described in the above <Analysis Method of Repeating α-1,6 Linkage Content in Reaction Product>. Subsequently, the proportion of α-1,6 linkages in the pentasaccharide component were analyzed by H-NMR according to the procedure of <Structural Analysis of Reaction Product>.

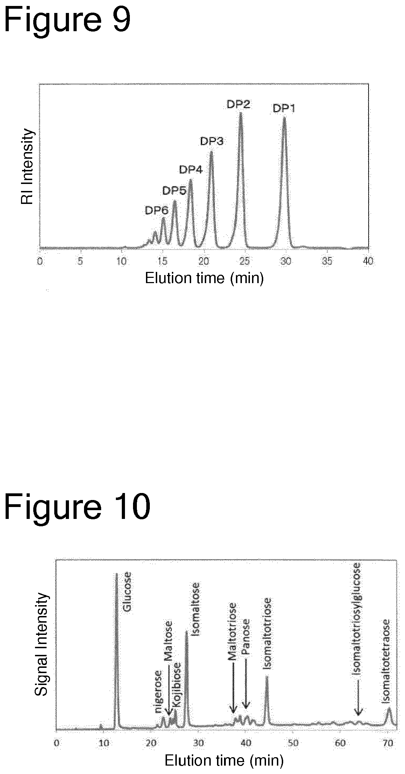

The chromatogram of the distribution of the degree of polymerization of the reaction production was shown in Figure 9, and the chromatogram of the isomer composition was shown in Figure 10.

The time courses of the molecular weight distribution of the reaction products until 72 h. was shown in Figure 11A, and the time courses of the isomer compositions of di-, tri-and tetra-saccharides was shown in Figure 11B. In the distribution of degree of polymerization, trisaccharide components were generated with the decrease of the substrate maltose (DP2), then, the components having an even higher degree of polymerization increase. In the isomer composition of the di-, tri- and tetra-saccharide components, α-1,4•1,6 linkage oligosaccharides and α-1,3 linkage oligosaccharide were increased at the initial stage of reaction, then turned decrease. Contrary, α-1,6 linkage oligosaccharides were increased from the initial stage to the middle stage of reaction, and reached equilibrium and maintained as it is.

The distribution of the molecular amount at 72 h. from the beginning was monosaccharide 27.9%, disaccharide 25.6%, trisaccharide 18.4%, and tetrasaccharide or higher 28.1%. Furthermore, among the α-1,4•1,6 linkage oligosaccharides and the α-1,6 linkage oligosaccharides, isomaltose which is a disaccharide existed in an amount of 17.8%, panose and isomaltotriose, which are trisaccharides, respectively in amounts of 2.7% and 10.8%, and isomaltotriosyl glucose and isomaltotetraose, which are tetrasaccharides, respectively in amounts of 1.0% and 7.8%.

Time courses of the compositions of tetrasaccharides or higher saccharides containing repeating α-1,6 linkages until 72 h was shown in Figure 12. Tetrasaccharides or higher saccharides containing repeatingα-1,6 linkages were increased to 14% until 72 h. This content of repeating α-1,6 linkages in tetrasaccharide or a higher saccharide was significantly high by comparison of a case, transglucosidase L "Amano" or the crude enzyme solution derived from an *Aspergillus niger* ATCC10254 strain was reacted with maltose.

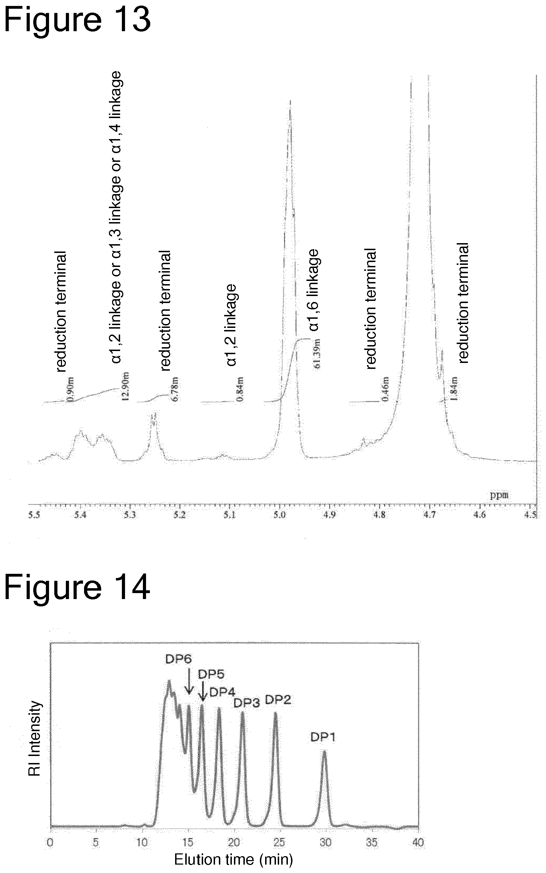

The purified enzyme of the present invention was reacted for 72 h. with maltose under the same reaction condition as mentioned above, and pentasaccharides were separated from the reaction product. This pentasaccharides were analyzed by H-NMR and showed that α-1,6 linkages were contained in an amount of 81% (Figure 13). In addition, much of the di-, tri- and tetra-saccharide components were consist of α-1,6 linkages (α-1,6 linkage oligosaccharides) as described above, and components of hexasaccharide or a higher saccharide are presumed to have a high proportion of α-1,6 linkages as well. In view of the above, most of the saccharide produced by reacting the enzyme of the present invention with maltose is connected by the α-1,6 linkage.

### <Transglycosylation Product of Maltopentaose (DP5)>

A purified enzyme (0.2 mL (7.2 U/mL) obtained by the method shown in 5) was added to 0.4 mL of a 45% maltopentaose solution (final concentration 30%) and incubated at 40°C. After a predetermined time passed, the solution was heated in the boiling bath for 10 min. to inactivate enzyme.

The distribution of the degree of polymerization of the obtained reaction product was analyzed according to the methods described in the above <Analysis Method of Distribution of Degree of Polymerization of Reaction Products>. Further, the content of repeating α-1,6 linkages in the reaction product was analyzed according to the method described in the above <Analysis Method of Repeating α-1,6 Linkage Content in Reaction Product>. In this analysis, the reduction in the proportion of components of hexasaccharide and a higher saccharide by dextranase treatment was interpreted as the content of α-1,6 linkages in hexasaccharide or a higher saccharide, and time course of the products were evaluated.

The chromatogram of the distribution of the degree of polymerization of the reaction production was shown in Figure 14.

It was also performed with transglucosidase L "Amano" or the crude enzyme solution derived from *Aspergillus niger* ATCC10254 strain in the same reaction and analyzed.

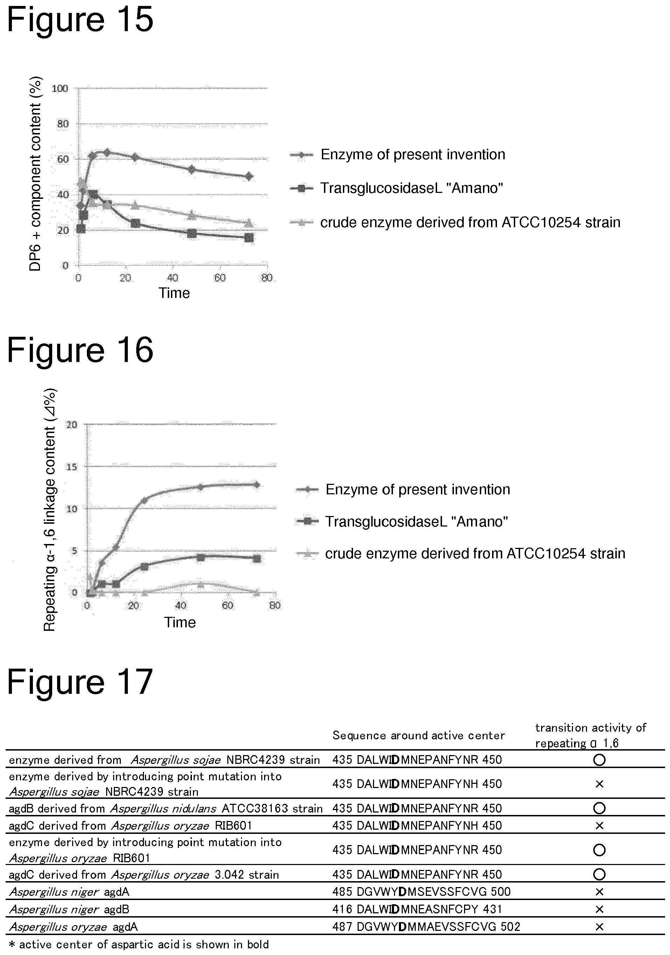

Time courses of the compositions of hexasaccharides or higher saccharides containing repeating α-1,6 linkages until 72 h. was shown in Figure 15. The content of hexasaccharide or a higher saccharide, it was obtained by the reaction of the enzyme of the present invention with maltopentaose, was significantly high by comparison of a case, transglucosidase L "Amano" or the crude enzyme solution derived from an *Aspergillus niger* ATCC10254 strain was reacted same condition.

Time courses of the compositions of hexasaccharides or higher saccharides containing repeating α-1,6 linkages was shown in Figure 16. The content of repeating α-1,6 linkages in hexasaccharide or a higher saccharide was increased to 13%. until 72 h. This content of repeating α-1,6 linkages in hexasaccharide or a higher saccharide was significantly high by comparison of a case, transglucosidase L "Amano" or the crude enzyme solution derived from an *Aspergillus niger* ATCC10254 strain was reacted with maltopentaose.

Methylation analysis of the hexasaccharide or a higher saccharide, the saccharide was separated from the reaction product obtained by the purified enzyme of the present invention was reacted with maltopentaose for 72 h. under the same reaction condition as above, was shown in Table 4. Other than the glucose of non-reduced terminals, proportion of the glucose constituting the reaction product was 62% for α-1,6 linkages and 30% for limited α-1,4 linkages. The enzyme of the present invention scarcely has an α-1,4 transglycosylation activity by the analysis of the reaction product that is obtained by reacting the enzyme of present invention to maltose. Therefore, it is considered that the α-1,4 linkages contained in the reaction product of the above was derived from original substrate of maltopentaose, and the bonds newly generated from the tranglycosylation reaction was mostly α-1,6 linkages.

**[Table 4]**

| Linkage Forms | |
|---|---|
| Structure | Compositional Ratio (non-reduced terminal is 1.00) |
| Glc 1 →(non-reduced terminal) | 1.00 |
| →3 Glc 1 → | 0.30 |
| →4 Glc 1 → | 1.55 |
| →6 Glc 1 →(*α*-1,6 linkage) | 2.76 |
| →2,4 Glc 1 → | 0.14 |
| →4,6 Glc 1 →(*α*-1,4,6 linkage) | 0.18 |
| →3,6 Glc 1 →(*α*-1,3,6 linkage) | 0.27 |
| Others | -- |

### 8) Preparation of α-Glucosidase Derived from Other Origin of the Genus Aspergillus and Evaluation Thereof

Gene recombinant strains were constructed to obtain crude enzyme other than those derived from the *Aspergillus sojae* NBRC4239 strain, namely, agdB (accession number EAA63748) derived from *Aspergillus nidulans* ATCC38163 strain and agdC derived from *Aspergillus oryzae* RIB601 strain, by the same procedure as that described in 4).
In addition, gene recombinant strains were obtained by transforming the arginine at the 450^{th} position of the amino acid sequence of an enzyme derived from the *Aspergillus sojae* NBRC4239 strain to histidine, and by transforming the histidine at the 450^{th} position of the amino acid sequence of an enzyme derived from the *Aspergillus oryzae* RIB601 strain to arginine, using the QuikChange Site-Directed Mutagenesis Kit (produced by Stratagene).

These crude enzyme solutions were subjected to reaction with maltopentaose by the same method as that in 7) <Transglycosylation Product of Maltopentaose (DP5)>, and the repeating α-1,6 linkage content of the reaction product was analyzed. The repeating α-1,6 linkage content in the hexasaccharides or higher saccharide of the reaction product after 24 h. was 8% for agdB derived from the *Aspergillus nidulans* ATCC38163 strain, 10% for the mutated enzyme of agdC derived from the *Aspergillus oryzae* RIB601 strain having a point mutation, and these strains produced about the same level of repeating α-1,6 linkages as the *Aspergillus sojae* NBRC4239 strain (11%). Contrary, agdC derived from the *Aspergillus oryzae* RIB601 strain and the mutated enzyme of *Aspergillus sojae* NBRC4239 having a point mutation, showed α-glucosidase activity, but produced no repeating α-1,6 linkages. The latter two types of enzymes were subjected to reaction with maltose, and the isomer composition was analyzed for the di-, tri- and tetra-saccharide components by the <Analysis Method of Isomer Composition of Reaction Product>. The result was that the content of oligosaccharides containing α-1,6 linkages was low, and much oligosaccharides containing α-1,3 linkages or α-1,4 linkages were produced.

### 9) Comparison of Sequences of α-Glucosidase Derived from the Genus Aspergillus

Enzymes belonging to the GH31 families are known to have highly conserved sequences in the active center (Plant Molecular Biology, vol. 37, p. 1, 1998, PROSITE:PS00129). Figure 17 shows the sequence from the conserved sequence near the active center to three residues downstream in sequences of various α-glucosidase derived from the genus *Aspergillus* and whether each α-glucosidase has a repeating α-1,6 transition activity. In the enzymes having repeating α-1,6 transition activity, all of the amino acids in the conserved sequence was matched. agdC derived from the *Aspergillus oryzae* RIB601 strain, which has the same conserved sequence but comprises histidine instead of arginine as the residue that is 10 residues downstream from the active center, had no repeating α-1,6 transition activity. The enzyme of the *Aspergillus sojae* NBRC4239 strain, which has the residue that is 10 residues downstream of the active center was changed from arginine to histidine by point mutation, also had no repeating α-1,6 transition activity. Other known enzymes, which are known to be α-glucosidase but not repeating α-1,6 transition enzymes, had different amino acids in the conserved sequence. Therefore, the sequence near the active center comprises "DALWIDMNEPANF" and the residue that is 10 residues downstream from the active center is other than histidine, are important for repeating α-1,6 transition activity.

In other words, an α-glucosidase that contains an amino acid sequence satisfying the above conditions, a sequence consisting of DALWIDMNEPANFX₁X₂X₃, wherein X₁X₂ is any amino acid and X₃ is an amino acid other than histidine, and that exhibits an activity of producing saccharide containing repeating α-1,6 linkages was discovered from the enzymes derived from *Aspergillus sojae, Aspergillus nidulans,* and mutated enzymes derived from *Aspergillus oryzae* RIB601 strains. Also, the *Aspergillus oryzae* 3.042 strain and the *Aspergillus flavus* NRRL3357 strain include sequences that are predicted as belonging to an enzyme of the GH31 family (respectively assigned Accession no. EIT77388 and Accession no. XP_002380576). The amino acid sequences of these enzymes have 98% sequence identity with the amino acid sequence shown by SEQ ID NO: 3, and they are presumed to produce enzymes that exhibit an activity for producing saccharide containing repeating α-1,6 linkages. *Aspergillus oryzae* and *Aspergillus flavus* are fungus belonging to the genus *Aspergillus* section *Flavi* similar to *Aspergillus sojae.* The database shows that *Penicillium chrysogenum, Nectria haematococca, Stachybotrys chlorohalonata, Stachybotrys chartarum, Fusarium oxysporum, Fusarium fujikuroi,* and *Fusarium verticillioides* also contain an enzyme of the GH31 family having a sequence "DALWIDMNEPANF" near the active center and a residue that is 10 residues downstream of the active center which is not histidine. These filamentous fungi have a high possibility of producing an enzyme that contains repeating α-1,6 transition activity similar to the α-glucosidase of the present invention.

The identity of the sequences of α-glucosidase derived from various *Aspergillus* were shown in Table 5. by comparing the amino acid sequence (SEQ ID NO: 3), genome gene sequence (SEQ ID NO: 1) and cDNA sequence (SEQ ID NO: 2) of α-glucosidase derived from the *Aspergillus sojae* NBRC4239. Furthermore, the genome sequence of the *Aspergillus oryzae* RIB601 had not been analyzed, the sequence of agdC derived from this strain was analyzed independently by the present inventors (SEQ ID NO: 4). The sequence identity was evaluated using the clustalW (http://www.genome.jp/tools/clustalw/) program.

**[Table 5]**

| α-glucosidase | Amino acid seq. | Genome gene seq. | cDNA seq. |
|---|---|---|---|
| enzyme derived from *Aspergillus sojae* NBRC4239 strain | 100% | 100% | 100% |
| enzyme derived from *Aspergillus nidulans* ATCC38163 strain | 82% | 77% | 78% |
| agdC derived from *Aspergillus orvzae* RIB40 | 98% | 93% | 94% |
| agdC derived from Aspergillus oryzae 33.042 strain | 98% | 93% | 94% |
| *Aspergillus niger* agdB | 53% | 54% | 58% |
| *Aspergillus niger* agdA | 31% | 9% | 23% |
| *Aspergillus oryzae* agdA | 30% | 2% | 6% |

### 10) Production Method of Enzymes

### <Production of α-Glucosidase Enzyme Solution Derived from the NBRC4239 Strain>

10 g of wheat bran was inserted to an 500ml of Erlenmeyer flask, followed by vapor sterilization thereof, then, 10 mL of preculture solution that is prepared by cultivating the *Aspergillus sojae* NBRC4239 strain in the YPD culture medium was added and the mixture was subjected to agitation to homogenize the water, followed incubated as a solid cultivation at 30°C for 4 days. After the cultivation process had ended, the wheat bran was washed with 100 mL of MES buffer (pH 6.0) and filtered with Miracloth (produced by Merck Millipore Corporation), and centrifuged at 3,500 rpm for 20 min., after the centrifugation process was repeated twice, the supernatant was collected to provide a crude enzyme solution. The enzyme activity of the obtained crude enzyme solution was evaluated by a method described in <Analysis Method of α-Glucosidase Activity>. The result showed an activity of 24U.

The obtained crude enzyme solution was subjected to reaction with maltose and maltopentaose by the respective methods of 7) <Transglycosylation Product of Maltose (DP2) derived from NBRC4239 Strain> and <Transglycosylation Product of Maltopentaose (DP5)>, and the content of the repeating α-1,6 linkages in the reaction product was analyzed by the method in <Analysis Method of Repeating α-1,6 Linkage Content in Reaction Product>. The saccharide containing repeating α-1,6 linkages was obtained.

## Claims

1. α-Glucosidase from a genus *Aspergillus,* and that comprises enzymologic features shown below:
(1) Action: react with saccharide selected from a group consisting of α-oligosaccharides and α-polysaccharides to form saccharide containing repeating α-1,6 linkages;
(2) Substrate specificity: acts on maltose, isomaltose, maltotriose, isomaltotriose, maltotetraose, isomaltotetraose, maltopentaose, maltohexaose, maltoheptaose, amylose, soluble starch, dextran.

2. The α-glucosidase according to claim 1 that further comprises enzymologic features shown below:
(3) Molecular amount: 65,000 dalton and 76,000 dalton according to SDS-polyacrylamide gel electrophoresis;
(4) Isoelectric point: pI 4.9 to 5.5 (central value 5.2)
(5) Optimum temperature: 55°C for a 30 min. reaction at pH 6.0;
(6) Optimum pH: pH 5.5 for a 30 min. reaction at 40°C;
(7) Temperature stability: 90% or more of initial activity remains after incubated in pH5.0 at 50°C for 1hr.;
(8) pH stability: 90% or more of initial activity remains after incubated in pH6.0 to 10.0 at 4°C for 24h.

3. The α-glucosidase according to either claim 1 or 2 that is either (a) or (b) below:
(a) α-glucosidase that consists of an amino acid sequence having at least 80% sequence identity with an amino acid sequence shown by SEQ ID NO: 3;
(b) α-glucosidase that consists of an amino acid sequence in which 1 to 190 amino acids are lost, substituted or added to an amino acid sequence shown by SEQ ID NO: 3.

4. α-Glucosidase that is either (a) or (b) below:
(a) α-glucosidase that consists of an amino acid sequence having at least 80% sequence identity with an amino acid sequence shown by SEQ ID NO: 3, and that react with saccharide selected from a group consisting of α-oligosaccharides and α-polysaccharides to form saccharide containing repeating α-1,6 linkages;
(b) α-glucosidase that consists of an amino acid sequence in which 1 to 190 amino acids are lost, substituted or added to an amino acid sequence shown by SEQ ID NO: 3, and that react with saccharide selected from a group consisting of α-oligosaccharides and α-polysaccharides to form saccharide containing repeating α-1,6 linkages.

5. α-Glucosidase that was collected from a culture obtained by cultivating genus Aspergillus having nucleic acids of one of (a) to (e) below:
(a) a nucleic acid containing a base sequence encoding protein that consists of an amino acid sequence having at least 80% sequence identity with an amino acid sequence shown by SEQ ID NO: 3, and that exhibits an activity of react with saccharide selected from a group consisting of α-oligosaccharides and α-polysaccharides to form saccharide containing repeating α-1,6 linkages;
(b) a nucleic acid containing a base sequence encoding protein that consists of an amino acid sequence in which 1 to 190 amino acids are lost, substituted or added to an amino acid sequence shown by SEQ ID NO: 3, and that exhibits an activity of react with saccharide selected from a group consisting of α-oligosaccharides and α-polysaccharides to form saccharide containing repeating α-1,6 linkages;
(c) a nucleic acid that hybridizes with a nucleic acid having of a base sequence that is complementary to a base sequence encoding protein that consists of an amino acid shown by SEQ ID NO: 3 under a highly stringent condition, and that contains a base sequence encoding protein that exhibits an activity of react with saccharide selected from a group consisting of α-oligosaccharides and α-polysaccharides to form saccharide containing repeating α-1,6 linkages;
(d) a nucleic acid that hybridizes with a nucleic acid having of a base sequence that is complementary to a base sequence that consists of SEQ ID NO: 2, and that contains a base sequence encoding protein that exhibits an activity of react with saccharide selected from a group consisting of α-oligosaccharides and α-polysaccharides to form saccharide containing repeating α-1,6 linkages; and
(e) a nucleic acid that consists of a base sequence having at least 75% sequence identity with a base sequence consisting of SEQ ID NO: 2, and that contains a base sequence encoding protein that exhibits an activity of react with saccharide selected from a group consisting of α-oligosaccharides and α-polysaccharides to form saccharide containing repeating α-1,6 linkages.

6. A recombinant vector containing a nucleic acid that is one of (a) to (e) below:
(a) a nucleic acid containing a base sequence encoding protein that consists of an amino acid sequence having at least 80% sequence identity with an amino acid sequence shown by SEQ ID NO: 3, and that exhibits an activity of react with saccharide selected from a group consisting of α-oligosaccharides and α-polysaccharides to form saccharide containing repeating α-1,6 linkages.;
(b) a nucleic acid containing a base sequence encoding protein that consists of an amino acid sequence in which 1 to 190 amino acids are lost, substituted or added to an amino acid sequence shown by SEQ ID NO: 3, and that exhibits an activity of react with saccharide selected from a group consisting of α-oligosaccharides and α-polysaccharides to form saccharide containing repeating α-1,6 linkages.;
(c) a nucleic acid that hybridizes with a nucleic acid consisting of a base sequence that is complementary to a base sequence encoding protein that consists of an amino acid shown by SEQ ID NO: 3 under a highly stringent condition, and that contains a base sequence encoding protein that exhibits an activity of react with saccharide selected from a group consisting of α-oligosaccharides and α-polysaccharides to form saccharide containing repeating α-1,6 linkages.;
(d) a nucleic acid that hybridizes with a nucleic acid consisting of a base sequence that is complementary to a base sequence consisting of SEQ ID NO: 2 under a highly stringent condition, and that contains a base sequence encoding protein that exhibits an activity of acting on sugar selected from a group consisting of α-oligosaccharides and α-polysaccharides to generate sugar containing repeating α-1,6 linkages;
(e) a nucleic acid that consists of a base sequence having at least 75% sequence identity with a base sequence consisting of SEQ ID NO: 2, and that contains a base sequence encoding protein that exhibits an activity of react with saccharide selected from a group consisting of α-oligosaccharides and α-polysaccharides to form saccharide containing repeating α-1,6 linkages.

7. A transformant obtained by transferring the recombinant vector according to claim 6.

8. α-Glucosidase that was collected from a culture obtained by cultivating the transformant according to claim 7.

9. The α-glucosidase according to any one of claims 1 to 5 and 8 containing an amino acid sequence of sequence of DALWIDMNEPANFX₁X₂X₃, wherein X₁X₂ is any amino acid, and X₃ is an amino acid other than histidine, in an amino acid sequence encoding α-glucosidase.

10. The α-glucosidase according to claim 9, wherein X₁ is tyrosine and X₂ is asparagine.

11. The α-glucosidase according to any one of claims 1 to 5 and 8 to 10, wherein the saccharide has repeating α-1,6 linkages containing at least three repeating α-1,6 linkages.

12. The α-glucosidase according to any one of claims 1 to 5 and 8 to 11, wherein α-oligosaccharides are selected from a group consisting of maltose, kojibiose, nigerose, isomaltose, maltotriose, panose, isomaltotriose, maltotetraose, isomaltotetraose, maltopentaose, maltohexaose, maltoheptaose, α-cyclodextrine, β-cyclodextrine, and γ-cyclodextrine, and α-polysaccharides are selected from a group consisting of amylose, soluble starch, glycogen, and dextran.

13. The α-glucosidase according to any one of claims 1 to 5 and 8 to 12 produced from a fungus of genus *Aspergillus* that belongs to genus *Aspergillus* section *Flavi.*

14. The α-glucosidase according to claim 13 produced from *Aspergillus sojae* or *Aspergillus oryzae.*

15. A method for producing α-glucosidase that comprises a step of cultivating a fungus of genus *Aspergillus* that contains a nucleic acid of one of (a) to (e) below to obtain a culture, and a step of collecting α-glucosidase from the culture:
(a) a nucleic acid containing a base sequence encoding protein that consists of an amino acid sequence having at least 80% sequence identity with an amino acid sequence shown by SEQ ID NO: 3, and that exhibits an activity of react with saccharide selected from a group consisting of α-oligosaccharides and α-polysaccharides to form saccharide containing repeating α-1,6 linkages;
(b) a nucleic acid containing a base sequence encoding protein that consists of an amino acid sequence in which 1 to 190 amino acids are lost, substituted or added to an amino acid sequence shown by SEQ ID NO: 3, and that exhibits an activity of react with saccharide selected from a group consisting of α-oligosaccharides and α-polysaccharides to form saccharide containing repeating α-1,6 linkages;
(c) a nucleic acid that hybridizes with a nucleic acid consisting of a base sequence that is complementary to a base sequence encoding protein that consists of an amino acid shown by SEQ ID NO: 3 under a highly stringent condition, and that contains a base sequence encoding protein that exhibits an activity of react with saccharide selected from a group consisting of α-oligosaccharides and α-polysaccharides to form saccharide containing repeating α-1,6 linkages;
(d) a nucleic acid that hybridizes with a nucleic acid consisting of a base sequence that is complementary to a base sequence consisting of SEQ ID NO: 2 under a highly stringent condition, and that contains a base sequence encoding protein that exhibits an activity of react with saccharide selected from a group consisting of α-oligosaccharides and α-polysaccharides to form saccharide containing repeating α-1,6 linkages;
(e) a nucleic acid that consists of a base sequence having at least 75% sequence identity with a base sequence consisting of SEQ ID NO: 2, and that contains a base sequence encoding protein that exhibits an activity of react with saccharide selected from a group consisting of α-oligosaccharides and α-polysaccharides to form saccharide containing repeating α-1,6 linkages..

16. A method for producing α-glucosidase that comprises a step of cultivating a transformant that has been transformed by a recombinant vector containing a nucleic acid of one of (a) to (e) below to obtain a culture, and a step of collecting α-glucosidase from that culture:
(a) a nucleic acid containing a base sequence encoding protein that consists of an amino acid sequence having at least 80% sequence identity with an amino acid sequence shown by SEQ ID NO: 3, and that exhibits an activity of react with saccharide selected from a group consisting of α-oligosaccharides and α-polysaccharides to form saccharide containing repeating α-1,6 linkages;
(b) a nucleic acid containing a base sequence encoding protein that consists of an amino acid sequence in which 1 to 190 amino acids are lost, substituted or added to an amino acid sequence shown by SEQ ID NO: 3, and that exhibits an activity of react with saccharide selected from a group consisting of α-oligosaccharides and α-polysaccharides to form saccharide containing repeating α-1,6 linkages;
(c) a nucleic acid that hybridizes with a nucleic acid consisting of a base sequence that is complementary to a base sequence encoding protein that consists of an amino acid shown by SEQ ID NO: 3 under a highly stringent condition, and that contains a base sequence encoding protein that exhibits an activity of react with saccharide selected from a group consisting of α-oligosaccharides and α-polysaccharides to form saccharide containing repeating α-1,6 linkages;
(d) a nucleic acid that hybridizes with a nucleic acid consisting of a base sequence that is complementary to a base sequence consisting of SEQ ID NO: 2 under a highly stringent condition, and that contains a base sequence encoding protein that exhibits an activity of react with saccharide selected from a group consisting of α-oligosaccharides and α-polysaccharides to form saccharide containing repeating α-1,6 linkages;
(e) a nucleic acid that consists of a base sequence having at least 75% sequence identity with a base sequence consisting of SEQ ID NO: 2, and that contains a base sequence encoding protein that exhibits an activity of react with saccharide selected from a group consisting of α-oligosaccharides and α-polysaccharides to form saccharide containing repeating α-1,6 linkages.

17. The method according to either claim 15 or 16 that contains a sequence of DALWIDMNEPANFX₁X₂X₃, wherein X₁X₂ is any amino acid, and X₃ is an amino acid other than histidine, in an amino acid sequence encoding α-glucosidase.

18. The method according to claim 17, wherein X₁ is tyrosine and X₂ is asparagine.

19. The method according to any one of claims 15 to 18, wherein the fungus of genus *Aspergillus* or the transformant is a fungus of genus *Aspergillus* that belongs to genus *Aspergillus* section *Flavi.*

20. The method according to claim 19, wherein the fungus of genus *Aspergillus* or the transformant is *Aspergillus sojae* or *Aspergillus oryzae.*

21. The method of producing sugar comprising applying α-glucosidase according to any one of claims 1 to 5 and 8 to 14 to sugar selected from a group consisting of an α-oligosaccharides and α-polysaccharides.
